# EUROPEAN PATENT APPLICATION

(11) **EP 3 146 981 A1**
(43) Date of publication of application: **29.03.2017**
(21) Application number: 15187139.9
(22) Date of filing: 28.09.2015
(51) Int. Cl.: A61K 47/50, C07K 1/13, A61P 43/00

(54) **COVALENTLY ATTACHED AZOBENZENE SWITCHES AND THEIR USES FOR THE OPTICAL CONTROL OF TARGET PROTEINS**

(71) Applicant: Ludwig-Maximilians-Universität München, 80539 München (DE)
(72) Inventor: Trauner, Dirk, 80539 Munich (DE); Broichhagen, Johannes, 81377 Munich (DE); Leippe, Philipp, 83101 Munich (DE); Sokol, Kevin Rafael, 80636 Munich (DE)
(74) Representative: Engelhard, Markus

(57) **Abstract**

The present invention relates to photo-switchable azobenzene compounds and their uses in the labeling of target proteins and as research tools. The present invention further relates to photo-sensor proteins, comprising a tag and the photo-switchable azobenzene compounds attached to said tag and their uses as research tools. The present invention further relates to the medical use of the photo-switchable azobenzene compounds and/or the photo-sensor proteins in the diagnosis and/or treatment of sight disorders and in the improvement of vision.

## Description

The present invention relates to photo-switchable azobenzene compounds and their uses in the labeling of target proteins and as research tools. The present invention further relates to photo-sensor proteins, comprising a tag and the photo-switchable azobenzene compounds attached to said tag and their uses as research tools. The present invention further relates to the medical use of the photo-switchable azobenzene compounds and/or the photo-sensor proteins in the diagnosis and/or treatment of sight disorders and in the improvement of vision.

### BACKGROUND OF THE INVENTION

The ability to covalently link synthetic molecules with proteins has significantly increased the power of molecular biology and has provided new therapeutic approaches *via* antibody drug conjugates. In recent years, chemical biologists have developed methods that can be used not only *in vitro* and in cell cultures, but can be also applied *in vivo*, even in large animals and, potentially, in humans (Hermanson 2013).

Important issues in bioconjugation are the speed, selectivity and orthogonality of the reaction and the extent to which the target protein needs to be modified to enable covalent attachment. Engineered cysteines have proved popular since they represent a minimal change in the protein structure and reliably react with certain electrophiles, such as maleimides (Hermanson 2013). More selective methods depend on the expansion of the genetic code and otherwise inert molecules that specifically react with protein motifs. These include self-labeling "tags", such as the SNAP-tag (Keppler et al., 2003; Keppler et al., 2004), the CLIP-tag (Gautier et al., 2008) or the Halo-tag (Los et al., 2008), and amino acid sequences that can be modified using external enzymes.

Bioconjugation has also played an important role in photopharmacology, which is an effort to control biological activity with synthetic photoswitches (Fehrentz et al., 2011). While soluble photochromic ligands (PCLs) are diffusion limited, photoswitchable tethered ligands (PTLs) covalently attach to an engineered site in the target protein. This places the ligand in the vicinity of its binding pocket, so that light maneuvers it in and out of a position where it can bind (Broichhagen and Trauner, 2014). The PTL approach allows for precise targeting since the bioconjugation motif, which is usually an engineered cysteine for maleimide conjugation, can be genetically encoded and selectively expressed in cells of interest. By contrast, PCLs act on native receptors, making for easier use, especially in therapeutic settings, albeit with less precision.

Although PTLs have proven to be powerful for controlling neural signaling and animal behavior (see e.g. Wyart et al., 2009; Levitz et al., 2013; Gaub et al., 2014), they have faced the disadvantages of cysteine/maleimide chemistry. Maleimides hydrolyze under physiological conditions and conjugate to glutathione, making them incompatible with the intracellular environment. Moreover, both in the cell and on the cell surface, they are likely to react with accessible native cysteines that are not at the designed PTL anchoring site. Although attachment to the introduced cysteine can be enhanced by affinity labeling due to increased times of proximity when the ligand binds in the binding pocket (Gorostiza et al., 2007), the susceptibility of maleimides to unwanted nucleophiles, including water, makes them less than ideal for applications in photopharmacology.

A solution to these challenges could be the introduction of electrophiles that react with very high selectivity and yet are stable toward water. Under normal circumstances, this requires a larger protein tag, moving the site of attachment far away from the ligand-binding site, typically in the range of several nanometers. Although tethers with photoswitches placed in series could be designed, multiple isomerization states of the tether and the spread of conformations of the long entropic spring (Schafer et al., 2007) could complicate control and prevent clean changes in biological activity upon irradiation.

There is a need in the art for improved photo-switchable compounds, which are in particular suitable for *in vivo* applications for the labeling proteins of interest, such as receptors, and for improved photo-sensor proteins.

### SUMMARY OF THE INVENTION

According to the present invention this object is solved by a photo-switchable azobenzene compound having the general formula I

**TAG** - **link** -**Azo** - **L** (I)

wherein
**TAG** is a moiety selected from a benzyl guanine (BG), benzyl cytosine (BC), maleimide (M) or alkyl halide (RX),
**link** is a linker group,
**Azo** is the azobenzene moiety, and
**L** is a binding moiety to a target protein.

According to the present invention this object is solved by a pharmaceutical composition, comprising
(i) at least one photo-switchable azobenzene compound according to the present invention,
(ii) optionally, pharmaceutically acceptable excipient(s) and/or carrier(s),
(iii) optionally, further compound(s).

According to the present invention this object is solved by a photo-sensor protein, comprising a tag for attaching the photo-switchable azobenzene compound according to the present invention, and
the photo-switchable azobenzene compound according to the present invention, which has been attached to the protein via said tag.

According to the present invention this object is solved by the use of the photo-switchable azobenzene compound according to the present invention for labeling of target proteins, wherein the target protein comprises a tag for labeling which is preferably selected from a SNAP-tag, a CLIP-tag, or a Halo tag.

According to the present invention this object is solved by the use of use of a photo-switchable azobenzene compound according to the present invention or a photo-sensor protein of the present invention as research tool,
such as:
- for the diagnosis or restoration of vision, sight disorder(s),
- the elucidation of signaling pathways,
- the mapping of neural circuitry,
- the activation/surpression of immune responses,
- the control of nociception
- the control of insulin release and other hormonal pathways, and/or
- as research tool(s) in general.

According to the present invention this object is solved by the photo-switchable azobenzene compound of the present invention or the pharmaceutical composition of the present invention or the photo-sensor protein of the present invention for use in medicine.

According to the present invention this object is solved by the photo-switchable azobenzene compound of the present invention or the pharmaceutical composition of the present invention or the photo-sensor protein of the present invention for use
- in the diagnosis and/or treatment of a sight disorder,
- in the improvement of vision,
- in the improvement of neural disorders,
- in the up-/downregulation of the immune system,
- in the treatment of pain sensation, i.e. nociception, and/or
- in the treatment of hormonal disorders, e.g. diabetes.

### DESCRIPTION OF THE PREFERRED EMBODIMENTS OF THE INVENTION

Before the present invention is described in more detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art. For the purpose of the present invention, all references cited herein are incorporated by reference in their entireties.

### Photo-switchable azobenzene compounds

Here we introduce a new concept, termed PORTL (Photoswitchable Orthogonal Remotely Tethered Ligand), that overcomes the limitations of maleimide chemistry and lays to rest concerns about off-target attachment (Figure 1). Like a PTL, a PORTL consists of a bioconjugation handle, a photoswitchable group, and a ligand. In this case, however, the switch does not primarily impact the overall length, pointing angle and flexibility of the tether, but rather the pharmacology of the tethered ligand. As such, the switch becomes an integral part of the pharmacophore and the change in biological activity is designed to result not from a change in the relative position of the ligand with respect to its binding site, but rather from a change in the efficacy of the ligand, as it does in a PCL. Therefore, the tether can be long and flexible, allowing for the use of larger bioconjugation motifs, such as a SNAP-tag, which provide an anchoring site at a more remote location with respect to the ligand-binding site. The SNAP-tag is a modified DNA repair enzyme that functions as a self-labeling domain by selectively and quickly reacting with benzylguanine (**BG**) electrophiles (Keppler et al., 2003; Keppler et al., 2004). It enables specific and efficient labeling with BG fluorophores in cultured cells and in brain slice (Reymond et al., 2011; Yang et al., 2015). Importantly, unlike maleimides, BGs are essentially inert toward water, regular cysteines and glutathione making them ideal for labeling in physiological systems that include extracellular and intracellular targets (Keppler et al., 2004; Juillerat et al., 2003; Bojkowska et al., 2011).

As discussed above, the present invention provides photo-switchable azobenzene compounds.

Said compounds have the general formula I

**TAG - link -Azo - L** (I)

wherein
**TAG** is a moiety selected from
a benzyl guanine (**BG**),
benzyl cytosine (**BC**),
maleimide (**M**) or
alkyl halide (**RX**),
**link** is a linker group,
**Azo** is the azobenzene moiety, and
**L** is a binding moiety to a target protein.

Preferably, the binding moiety to the target protein **L** is selected from
- a substrate, an inhibitor, a cofactor, an agonist, an antagonist, a modulator of the target protein,
- a ligand of a receptor of interest (such as an agonist, antagonist, inhibitor, blocker or modulator),
   preferably a ligand of a G-protein coupled receptor (GPCR), an ionotropic receptor and/or a receptor-linked enzyme
   such as a glutamate, a tetraalkyl ammonium moiety or other pharmaceutical group.

Preferably, the azobenzene moiety **Azo** undergoes *trans-cis* isomerization by excitation with light, or by illumination and dark relaxation.

In particular, the azobenzene moiety undergoes *cis*-isomerization by excitation with light and *trans*-isomerization by illumination and/or dark relaxation.

This intrinsic property can be used to induce a conformational change in the molecule and change binding affinity/efficacy towards a biological target accordingly.

In a preferred embodiment, the azobenzene moiety **Azo** is an azobenzene or a derivative thereof.

Examples for azobenzene derivatives are:
- heterocyclic azobenzene structure(s),
- substituted azobenzene structure(s) (e.g. tetra-ortho),
- bridged azobenzenes (e.g. by an o,o'-ethylene chain).

Preferably, the activation wavelength is in the ultraviolet, visible and infrared light ranges of the electromagnetic spectrum.

Preferably, the linker group **link** is a polyethylene glycol (PEG).

In a preferred embodiment, the photo-switchable azobenzene compound of the present invention has the general formula Ia

**BG - (link)ₙ -Azo - glu** (**Ia**)

wherein
**BG** is the benzyl guanine moiety,
**link** is a linker group, preferably polyethylene glycol with **n** being 0 to 12, **Azo** is the azobenzene moiety, and
**glu** is a glutamate group.

In a preferred embodiment, the photo-switchable azobenzene compound of the present invention has one of the formulas or is selected from
**BGAGₙ** 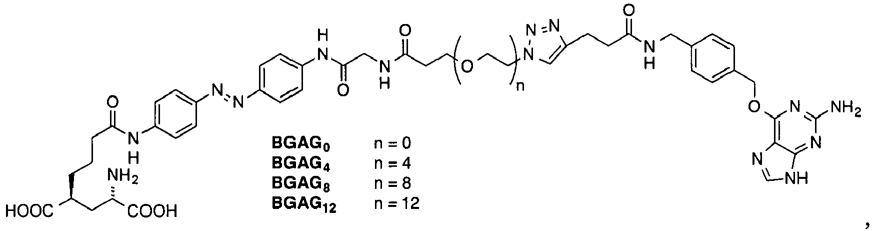 **BGAG₀:** (2*S*,4*S*)-2-Amino-4-(4-((4-((*E*)-(4-(2-(3-(4-(3-((4-(((2-amino-9*H*-purin-6-yl)oxy)methyl) benzyl)amino)-3-oxopropyl)-1*H*-1,2,3-triazol-1-yl)propanamido)acetamido)-phenyl)diazenyl)phenyl)amino)-4-oxobutyl)pentanedioic acid;
**BGAG₄:** (2S,4S)-2-Amino-4-(4-((4-((*E*)-(4-(1-(4-(3-((4-(((2-amino-9H purin-6-yl)oxy) methyl)-benzyl)amino)-3-oxopropyl)-1*H*-1,2,3-triazol-1-yl)-15-oxo-3,6,9,12-tetraoxa-16-azaoctadecan-18-amido)phenyl)diazenyl)phenyl)amino)-4-oxobutyl)-pentanedioic acid,
**BGAG₈:** (2S,4S)-2-Amino-4-(4-((4-((E)-(4-(1-(4-(3-((4-(((2-amino-9*H*-purin-6-yl)oxy) methyl)-benzyl)amino)-3-oxopropyl)-1*H*-1,2,3-triazol-1 -yl)-27-oxo-3,6,9,12,15,18,21,24-octa-oxa-28-azatriacontan-30-amido)phenyl)diazenyl)phenyl)amino)-4-oxobutyl)pentanedioic acid,
**BGAG₁₂**: (2S,4S)-2-Amino-4-(4-((4-((E)-(4-(1-(4-(3-((4-(((2-amino-9*H*-purin-6-yl)oxy) methyl)benzyl)amino)-3-oxopropyl)-1*H*-1,2,3-triazol-1-yl)-39-oxo-3,6,9,12,15,18,21,24,27,30,33,36-dodecaoxa-40-azadotetracontan-42-amido)phenyl)diazenyl)phenyl)amino)-4-oxobutyl)pentanedioic acid,
or **BGAG₁₂(₄₆₀):** (2*S*,4*S*)-2-amino-4-(4-((4-((*E*)-(4-((1-(8-(4-((4-(((2-amino-9H-purin-6-yl) oxy)methyl)-benzyl)amino)-4-oxobutanoyl)-8,9-dihydro-1*H*-dibenzo[b,f][1,2,3]triazolo[4,5-d]azo-cin-1-yl)-39-oxo-3,6,9,12,15,18,21,24,27,30,33,36-dodecaoxa-40-azadotetra-contan-42-yl)amino)phenyl)diazenyl)phenyl)amino)-4-oxobutyl)pentanedioic acid, 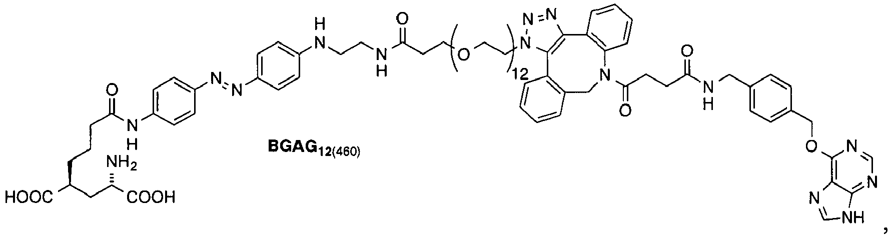 or **BGAQ₁₂:** (*E*)-2-((4-((4-(1-(4-(3-((4-(((2-ammo-9*H*-purin-6-yl)oxy)methyl)benzyl)amino)-3-oxopropyl)-1*H*-1,2,3-triazol-1-yl)-3,6,9,12,15,18,21,24,27,30,33,36-dodecaoxanonatriacontan-39-amido)phenyl)diazenyl)phenyl)amino)-*N*,*N*,*N*-triethyl-2-oxoethan-1-aminium 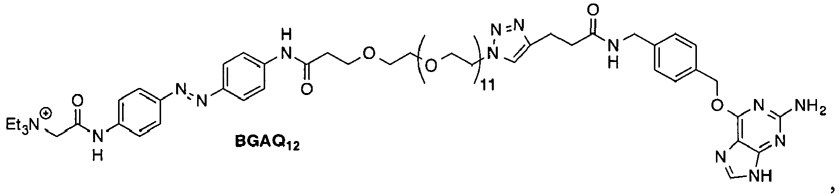 or ***L*-BGAG₁₂:** (2*S*,4*R*)-2-Amino-4-(4-((4-((*E*)-(4-(1-(4-(3-((4-(((2-amino-9*H*-purin-6-yl)oxy) methyl)benzyl)amino)-3-oxopropyl)-1*H*-1,2,3-triazol-1-yl)-39-oxo-3,6,9,12,15,18,21,24,27,30,33,36-dodecaoxa-40-azadotetracontan-42-amido)phenyl)diazenyl)phenyl)amino)-4-oxobutyl)pentanedioic acid 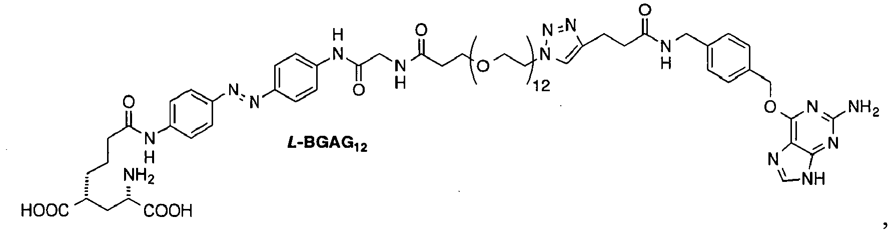 or **BCAG₁₂:** (2S,4S)-2-amino-4-(4-((4-((*E*)-(4-(1-(4-(3-((4-(((4-aminopyrimidin-2-yl)oxy)methyl)benzyl)amino)-3-oxopropyl)-1*H*-1,2,3-triazol-1-yl)-39-oxo-3,6,9,12,15,18,21,24,27,30,33,36-dodecaoxa-40-azadotetracontan-42-amido)phenyl)diazenyl)phenyl)amino)-4-oxobutyl)pentanedioic acid 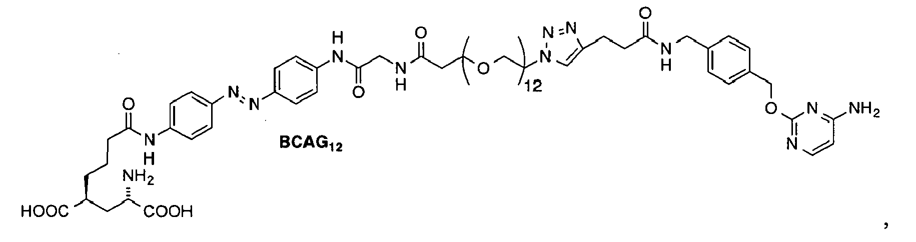 or **CIAG₁₂:** (2*S*,4*S*)-2-amino-4-(4-((4-((E)-(4-(1-(4-(3-((2-(2-((6-chlorohexyl)oxy)ethoxy)ethyl)amino)-3-oxopropyl)-1*H*-1,2,3-triazol-1-yl)-39-oxo-3,6,9,12,15,18,21,24,27,30,33,36-dodecaoxa-40-azadotetracontan-42-amido)phenyl)diazenyl)phenyl)amino)-4-oxobutyl)pentanedioic acid 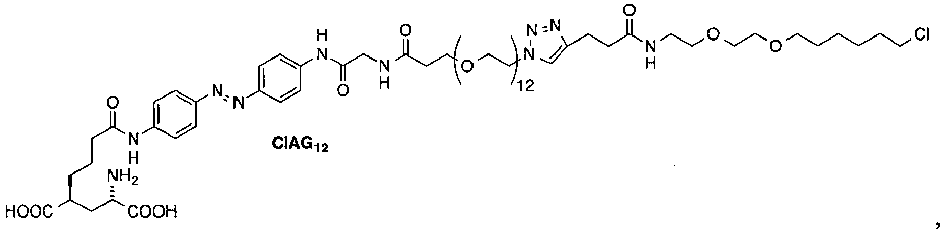 or **BGAG:** (2*S*,4*S*)-2-amino-4-(4-((4-((*E*)-(4-(3-(4-(3-((4-(((2-amino-9*H*-purin-6-yl)oxy)methyl)benzyl)amino)-3-oxopropyl)-1*H*-1,2,3-triazol-1-yl)propanamido)phenyl)-diazenyl)phenyl)amino)-4-oxobutyl)pentanedioic acid 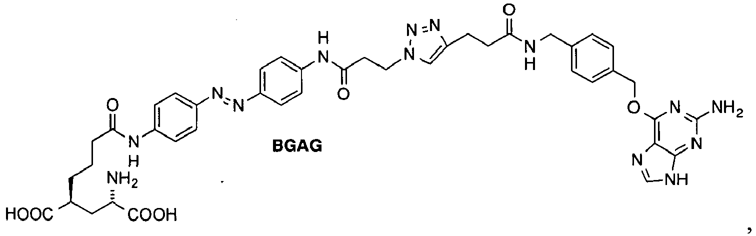 or **BGAP₁₂:** (*E*)-1-(4-(3-((4-(((2-amino-9*H*-purin-6-yl)oxy)methyl)benzyl)amino)-3-oxopropyl)-1*H*-1,2,3-triazol-1-yl)-*N*-(2-((4-((4-hydroxy-3,5-diisopropylphenyl)-diazenyl)phenyl)amino)ethyl)-3,6,9,12,15,18,21,24,27,30,33,36-dodecaoxanonatriacontan-39-amide 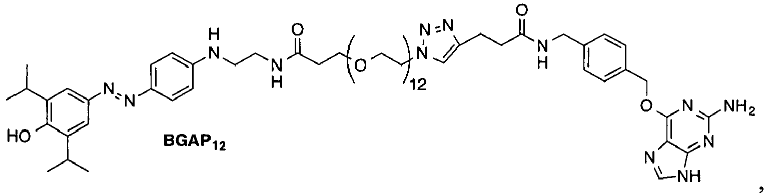 or **BGAF**₁₂: (*E*)-1-(4-(3-((4-(((2-amino-9*H*-purin-6-yl)oxy)methyl)benzyl)amino)-3-oxopropyl)-1*H*-1,2,3-triazol-1-yl)-*N*-(4-((4-(*N*-(1-phenethylpiperidin-4-yl)propionamido)-phenyl)diazenyl)phenyl)-3,6,9,12,15,18,21,24,27,30,33,36-dodecaoxanonatriacontan-39-amide 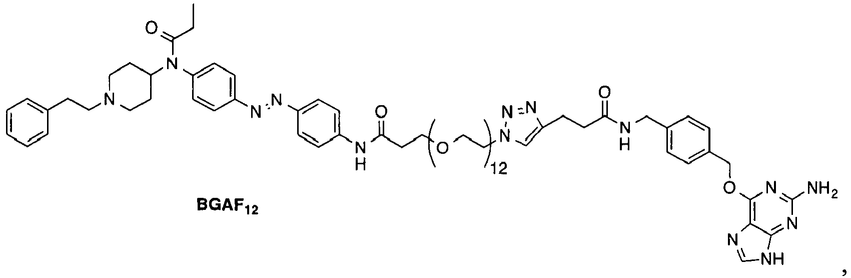 or **BGACh₁₂:** (*E*)-2-(4-((4-(1-(4-(3-((4-(((2-amino-9*H*-purin-6-yl)oxy)methyl)benzyl)amino)-3-oxopropyl)-1*H*-1,2,3-triazol-1-yl)-3,6,9,12,15,18,21,24,27,30,33,36-dodecaoxanonatriacontan-39-amido)phenyl)diazenyl)phenoxy)-*N,N,N-*trimethylethan-1-aminium 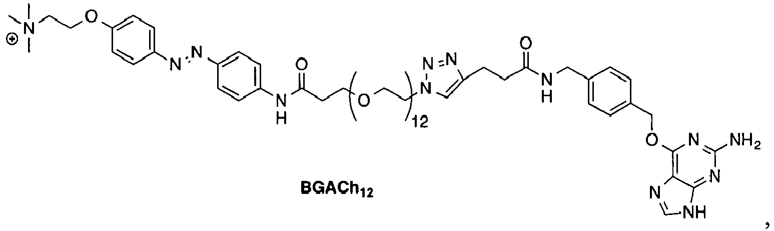 preferably **BGAG₁₂** or **BGAG**_{**12**(460)}**:**

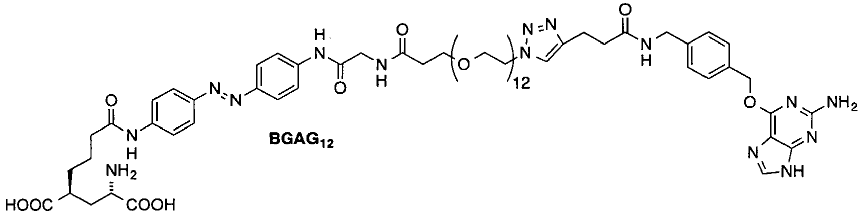

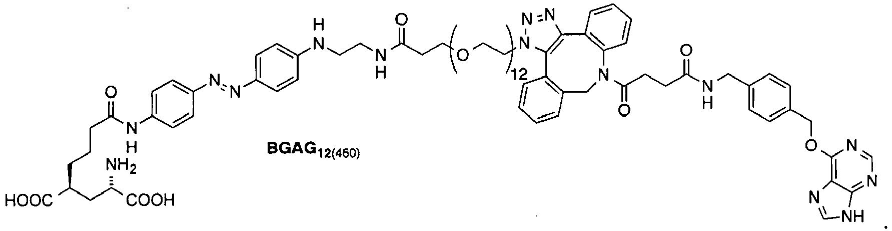

In one embodiment, the compound of the present invention can comprise further group(s) or moiety(ies), such as
- label (e.g. radioisotopes, MRI responsive groups, IR dyes, fluorophores)
- tag(s) (e.g. antibodies, nanobodies, peptides),
- anchoring group(s) (e.g. GPI),
   and/or
- other pharmacological units (e.g. fentanyl, GABA, choline, propofol).

As discussed above, the present invention provides a pharmaceutical composition, comprising
(i) at least one photo-switchable azobenzene compound according to the present invention,
(ii) optionally, pharmaceutically acceptable excipient(s) and/or carrier(s).

In an embodiment, the pharmaceutical composition of the present invention can comprise further compound(s) or drug(s).

Said further compound(s) or drug(s) can be used to optically remote-control important transmembrane proteins, such as muscarinic/nicotinic acetylcholine receptors, opioid receptors, GABA_{A/B} receptors and potassium channels.

### Photo-sensor proteins

As discussed above, the present invention provides a photo-sensor protein.

Said protein comprises
a tag for attaching the photo-switchable azobenzene compound according to the present invention, and
the photo-switchable azobenzene compound according to the present invention, which has been attached to the protein via said tag.

Said protein can further comprise a spacer between the tag and the protein and/or between the photo-switchable azobenzene compound and the tag.

Preferably, the photo-sensor protein is a transmembrane protein or receptor,
such as
a G protein coupled receptor (GPCR), e.g. a metabotropic glutamate receptor (mGluR2),
an ionotropic receptor,
ion channel and/or
a receptor-linked enzyme.

The tag is a tag which enables the attachment of the photo-switchable azobenzene compound of the present invention.

The tag is preferably selected from tags than can be introduced to the protein via cloning (to form respective fusions of tag and protein), such as a SNAP-tag, a CLIP-tag, or a Halo tag. SNAP- and CLIP-tag protein labeling systems enable the specific, covalent attachment of virtually any molecule to a protein of interest. There are two steps to using this system: cloning and expression of the protein of interest as a SNAP-tag® fusion, and labeling of the fusion with the SNAP-tag substrate of choice (in this case the photo-switchable azobenzene compound of the present invention).

For more information: https://www.neb.com/applications/protein-analysis-and-tools/protein-labeling/protein-labeling-snap-clip. See also e.g. Keppler et al., 2003; Keppler et al., 2004 (for SNAP-tag) or Gautier et al., 2008 (for CLIP-tag).

The SNAP-tag is a small protein based on human O⁶-alkylguanine-DNA-alkyltransferase (hAGT), a DNA repair protein. SNAP-tag substrates are dyes, fluorophores, biotin, or beads conjugated to guanine or chloropyrimidine leaving groups via a benzyl linker. In the labeling reaction, the substituted benzyl group of the substrate (i.e. the photo-switchable azobenzene compound of the present invention) is covalently attached to the SNAP-tag.

CLIP-tag™ is a modified version of SNAP-tag, engineered to react with benzylcytosine rather than benzylguanine derivatives. When used in conjunction with SNAP-tag, CLIP-tag enables the orthogonal and complementary labeling of two proteins simultaneously in the same cells.

HaloTag® technology is a tool for functional protein analysis based on the formation of a covalent bond between the HaloTag® protein fusion tag and synthetic chemical ligands (such as the photo-switchable azobenzene compound of the present invention). By interchanging ligands, one can control the function and properties of the HaloTag® fusion protein, allowing use of the same construct for protein purification, protein interaction analysis, and cell-based assays for protein translocation, receptor internalization, and protein stability studies.

For more information, see: http://www.promega.de/products/protein-purification-and-interactions/halotag-protein-purification/halotag-technology/. See also Los et al., 2008.

In a preferred embodiment, the photo-sensor protein of the present invention is a SNAP-tagged metabotropic glutamate receptor (SNAP-mGluR2), preferably labeled with **BGAG₁₂** or **BGAG**_{**12**(460)}.

### The rat metabotropic glutamate receptor mGluR2

### Uniprot Accession No. P31421

### Gene name: Grm2

### Amino acid sequence: [SEQ ID NO. 1]

10 20 30 40 50
MESLLGFLAL LLLWGAVAEG PAKKVLTLEG DLVLGGLFPV HQKGGPAEEC
60 70 80 90 100
GPVNEHRGIQ RLEAMLFALD RINRDPHLLP GVRLGAHILD SCSKDTHALE
110 120 130 140 150
QALDFVRASL SRGADGSRHI CPDGSYATHS DAPTAVTGVI GGSYSDVSIQ
160 170 180 190 200
VANLLRLFQI PQISYASTSA KLSDKSRYDY FARTVPPDFF QAKAMAEILR
210 220 230 240 250
FFNWTYVSTV ASEGDYGETG IEAFELEARA RNICVATSEK VGRAMSRAAF
260 270 280 290 300
EGVVRALLQK PSARVAVLFT RSEDARELLA ATQRLNASFT WVASDGWGAL
310 320 330 340 350
ESVVAGSERA AEGAITIELA SYPISDFASY FQSLDPWNNS RNPWFREFWE
360 370 380 390 400
ERFHCSFRQR DCAAHSLRAV PFEQESKIMF VVNAVYAMAH ALHNMHRALC
410 420 430 440 450
PNTTHLCDAM RPVNGRRLYK DFVLNVKFDA PFRPADTDDE VRFDRFGDGI
460 470 480 490 500
GRYNIFTYLR AGSGRYRYQK VGYWAEGLTL DTSFIPWASP SAGPLPASRC
510 520 530 540 550
SEPCLQNEVK SVQPGEVCCW LCIPCQPYEY RLDEFTCADC GLGYWPNASL
560 570 580 590 600
TGCFELPQEY IRWGDAWAVG PVTIACLGAL ATLFVLGVFV RHNATPVVKA
610 620 630 640 650
SGRELCYILL GGVFLCYCMT FVFIAKPSTA VCTLRRLGLG TAFSVCYSAL
660 670 680 690 700
LTKTNRIARI FGGAREGAQR PRFISPASQV AICLALISGQ LLIVAAWLVV
710 720 730 740 750
EAPGTGKETA PERREVVTLR CNHRDASMLG SLAYNVLLIA LCTLYAFKTR
760 770 780 790 800
KCPENFNEAK FIGFTMYTTC IIWLAFLPIF YVTSSDYRVQ TTTMCVSVSL
810 820 830 840 850
SGSVVLGCLF APKLHIILFQ PQKNVVSHRA PTSRFGSAAP RASANLGQGS
860 870
GSQFVPTVCN GREVVDSTTS SL

### The human metabotropic glutamate receptor mGluR2

### Uniprot Accession No. Q14416

### Gene name: GRM2

### Amino acid sequence: [SEQ ID NO. 2]

10 20 30 40 50

60 70 80 90 100

110 120 130 140 150

160 170 180 190 200

210 220 230 240 250

260 270 280 290 300

310 320 330 340 350


        360         370         380         390         400

        410         420         430         440         450

        460         470         480         490         500

        510         520         530         540         550

        560         570         580         590         600

        610         620         630         640         650

        660         670         680         690         700

        710         720         730         740         750

        760         770         780         790         800

        810         820         830         840         850

        860         870


We demonstrate the validity of the PORTL concept by fusing the class C G protein-coupled receptor (GPCR), mGluR2, with a SNAP-tag and endowing it with a synthetic azobenzene photoswitch through benzylguanine chemistry. The resulting photoreceptor, termed SNAG-mGluR2 (**SN**AP-tagged-**A**zobenzene-**G**lutamate receptor), permits rapid, repeatable, high-efficacy photoagonism of mGluR2 with thermally bistable and fast relaxing photoswitches. SNAG-mGluR2 allows for optical manipulation of neuronal excitability and synaptic transmission in hippocampal neurons. We also show that the SNAG approach may be combined with the cysteine attachment of a conventional PTL to allow for orthogonal optical control of two glutamate receptors within the same cell, paving the way for other multiplexing strategies.

### Uses of the photo -switchable azobenzene compounds and photo-sensor proteins

As discussed above, the present invention provides the use of the photo-switchable azobenzene compound according to the present invention for labeling of target proteins.

The target protein is preferably a transmembrane protein or receptor,
such as a G protein coupled receptor (GPCR), e.g. a metabotropic glutamate receptor (mGluR2).

The target protein comprises a tag for labeling which is preferably selected from a SNAP-tag, a CLIP-tag, or a Halo tag.

Preferably, the target proteins are labled *in vitro*, in intact tissue or *in vivo.*

As discussed above, the present invention provides the use of
- a photo-switchable azobenzene compound according to the present invention, or
- a photo-sensor protein of the present invention as research tool,
   such as:
   - for the diagnosis or restoration of vision, sight disorder(s),
   - the elucidation of signaling pathways,
   - the mapping of neural circuitry,
   - the activation/surpression of immune responses,
   - the control of nociception
   - the control of insulin release and other hormonal pathways, and/or
   - as research tool(s) in general.

Said use can comprise the use a further photo-sensor protein, such as a maleimide-based photo-sensor protein.

As discussed above, the present invention provides
- the photo-switchable azobenzene compound of the present invention, or
- the pharmaceutical composition of the present invention, or
- the photo-sensor protein of the present invention
   for use in medicine.

As discussed above, the present invention provides
- the photo-switchable azobenzene compound of the present invention, or
- the pharmaceutical composition of the present invention, or
- the photo-sensor protein of the present invention
   for use
   - in the diagnosis and/or treatment of a sight disorder,
   - in the improvement of vision,
   - in the improvement of neural disorders,
   - in the up-/downregulation of the immune system,
   - in the treatment of pain sensation, i.e. nociception, and/or
   - in the treatment of hormonal disorders, e.g. diabetes.

In one embodiment, the medical use comprises the orthogonal optical control of two receptors within the same cell.

### Further description of preferred embodiments

### - Abstract

The covalent attachment of synthetic photoswitches is a general approach to impart light-sensitivity onto native receptors. It mimics the logic of natural photoreceptors and significantly expands the reach of optogenetics. Here we describe a novel photoswitch design - the Photoswitchable Orthogonal Remotely Tethered Ligand (PORTL) - that combines the genetically encoded SNAP-tag with photochromic ligands connected to a benzyl guanine *via* a long flexible linker. We use the method to convert the G protein-coupled receptor mGluR2, a metabotropic glutamate receptor, into a photoreceptor (SNAG-mGluR2) that provides efficient optical control over the neuronal functions of mGluR2: presynaptic inhibition and control of excitability. The PORTL approach enables multiplexed optical control of different native receptors using distinct bioconjugation methods. It should be broadly applicable since SNAP-tags have proven to be reliable, many SNAP-tagged receptors are already available, and photochromic ligands on a long leash are readily designed and synthesized.

Here we introduce a new concept, termed PORTL (**P**hotoswitchable **O**rthogonal **R**emotely Tethered Ligand), that overcomes the limitations of maleimide chemistry and lays to rest concerns about off-target attachment (Figure 1). Like a PTL, a PORTL consists of a bioconjugation handle, a photoswitchable group, and a ligand. In this case, however, the switch does not primarily impact the overall length, pointing angle and flexibility of the tether, but rather the pharmacology of the tethered ligand. As such, the switch becomes an integral part of the pharmacophore and the change in biological activity is designed to result not from a change in the relative position of the ligand with respect to its binding site, but rather from a change in the efficacy of the ligand, as it does in a PCL. Therefore, the tether can be long and flexible, allowing for the use of larger bioconjugation motifs, such as a SNAP-tag, which provide an anchoring site at a more remote location with respect to the ligand-binding site. The SNAP-tag is a modified DNA repair enzyme that functions as a self-labeling domain by selectively and quickly reacting with benzylguanine (**BG**) electrophiles (Keppler et al., 2003; Keppler et al., 2004). It enables specific and efficient labeling with BG fluorophores in cultured cells and in brain slice (Reymond et al., 2011; Yang et al., 2015). Importantly, unlike maleimides, BGs are essentially inert toward water, regular cysteines and glutathione making them ideal for labeling in physiological systems that include extracellular and intracellular targets (Keppler et al., 2004; Juillerat et al., 2003; Bojkowska et al., 2011).

We demonstrate the validity of the PORTL concept by fusing the class C G protein-coupled receptor (GPCR), mGluR2, with a SNAP-tag and endowing it with a synthetic azobenzene photoswitch through benzylguanine chemistry. The resulting photoreceptor, termed SNAG-mGluR2 (**SN**AP-tagged-**A**zobenzene-**G**lutamate receptor), permits rapid, repeatable, high-efficacy photoagonism of mGluR2 with thermally bistable and fast relaxing photoswitches. SNAG-mGluR2 allows for optical manipulation of neuronal excitability and synaptic transmission in hippocampal neurons. We also show that the SNAG approach may be combined with the cysteine attachment of a conventional PTL to allow for orthogonal optical control of two glutamate receptors within the same cell, paving the way for other multiplexing strategies.

### - Discussion

Photoswitchable tethered ligands (PTLs) provide a powerful component of the optogenetic arsenal for biophysical, synaptic, neural circuit, behavioral and disease treatment applications (see e.g. Reiner et al., 2015). Unlike opsin-based approaches, which rely on the exogenous expression of non-native light-gated membrane proteins, PTLs offer target-specific control of native signaling proteins through the bioconjugation of synthetic light-controlled compounds. They allow one to study the physiological roles of individual proteins with a high subtype specificity and spatiotemporal and genetic precision compared to classical pharmacological or genetic techniques. Until the present, PTL anchoring to the signaling protein of interest has been almost exclusively based on the covalent attachment of a maleimide group on the PTL to an engineered cysteine positioned near the pore or ligand binding pocket of the protein (Lemoine et al., 2013). Even on extracellular parts of proteins, where most native cysteines are disulfide bonded and not subject to attack by a maleimide, there are many free cysteines where PTLs will attach. As a result, the specificity of action of cysteine-reactive PTLs relies not on unique targeting, but on the insensitivity of other proteins to the minor repositioning of tethered ligands (Volgraf et al., 2006; Lin et al., 2014).

Still, there would be a major advantage if protein attachment could be bio-orthogonal and so highly specific. Maleimide-cysteine attachment has proven viable in small animals, such as zebrafish and easily accessible tissues, such as the outer retina of mouse. However, it may be inefficient in larger systems due to slow diffusion and competition with hydrolysis, and is restricted to the extracellular environment, since inside the cell competition for the target cysteine by glutathione at millimolar concentrations would be forbidding. In addition, attachment to a native accessible cysteine, such as in enzyme active site, could be deadly. Our goal was to create a new orthogonal and efficient strategy for specific PTL attachment that is easy to generalize. We present a solution to these challenges in the form of a second generation PTL, termed PORTL, an approach built around the conjugation of BG-labeled photoswitches to genetically encoded SNAP tags.

The PORTL approach takes advantage of the fact that the SNAP-tag reacts with BGs in a very efficient and selective way that is fully orthogonal to native chemical reactions (Keppler et al., 2003; Keppler et al., 2004). Unlike first-generation PTLs, which need to be tethered near the site of ligand binding (see e.g. Banghart et al., 2004; Levitz et al., 2013; Fortin et al., 2008), PORTL tethers the photoswitch farther away, on a separate domain, providing a useful separation between the attachment point and functional head group of the compound by a long linker. In principle, the photoswitch could also be attached to a separate transmembrane protein, an antibody or a membrane anchor. This physical separation is expected to place the ligand head group of a PORTL at a relatively lower local concentration than a conventional PTL. The head group would then be photoswitched between active and active states like a photochromic ligand, and should be ideally inactive in the dark. Aspects of this logic were previously applied to a photoswitchable ligand attached via a long flexible tether to a GABA_{A} receptor, although in that case the ligand was a potentiator, not an agonist, the ligand was active in the dark, and the attachment was to an introduced cysteine (Yue et al., 2012). A further feature to our design is that the predicted relatively low local concentration of PORTL head groups may help ensure the lack of basal modulation of receptor activity by the relaxed state of the photoswitch.

With these considerations in mind, we designed and synthesized benzylguanine-azoglutamate (BGAG) PORTL compounds that may be attached to a SNAP-tagged version of the class C GPCR mGluR2 to produce the chemical optogenetic tool termed "SNAG-mGluR2". SNAG-mGluR2 permits the high-efficacy, rapid, repeatable photoactivation of mGluR2 with a 2-color, bistable BGAG (SNAG-mGluR2) or a 1-color, spontaneously relaxing **BGAG**_{**12**(460)} (SNAG₄₆₀-mGluR2). In both cases, SNAG-mGluR2 remains inactive in the dark and is activated in the high-energy state in response to either near UV (~380 nm, **BGAGₙ)** or visible light (~460 nm, **BGAG**_{**12**(460)}). Consistent with our predictions about the mechanism of PORTL photoactivation, untethered photoswitches that mimic the azobenzene-glutamate part of BGAG showed the same directionality of photoswitching on mGluR2, suggesting that the efficacy of the photoswitchable ligand is higher in *cis* than *trans* and independent of the tether. Importantly, since it maintains the entire full-length sequence of mGluR2, SNAG-mGluR2 should also retain all native signaling properties ranging from ligand binding to G protein coupling to downstream regulation. Consistent with this, SNAG-mGluR2 permitted efficient optical manipulation of two distinct native downstream targets of mGluR2 in neurons: a somato-dendritic control of excitability and a presynaptic control of synaptic transmission.

In line with the attractive properties of SNAP-tag conjugation, BGAG photosensitizes SNAP-mGluR2 at concentrations 100-1000x lower than typically used for maleimide-based PTLs, minimizing potential activation of glutamate receptors during photoswitch incubation. Furthermore, owing to its insensitivity to hydrolysis by water, BGAG remains reactive over not minutes but days, and stocks diluted in aqueous buffer may be re-used without a loss of labeling efficiency. Taken together, these properties should make the PORTL approach ideally suited for labeling in intact tissue or *in vivo,* as was recently shown for fluorophore conjugation to a SNAP-tag in the nervous system of mouse (Yang et al., 2015).

Another major advantage of the PORTL approach is its modularity, which will allow it to be widely applicable to many protein targets with a variety of photoswitches. The SNAP-tag is well characterized and has been used extensively to label fusion proteins with fluorophores or to create semisynthetic probes for the sensing of small molecules (Reymond et al., 2011). Like GFP, the SNAP tag can be fused to proteins of interest without significantly altering their activity. Indeed, several SNAP-tagged transmembrane class A and class C GPCRs, including all of the mGluRs (see e.g. Maurel et al., 2008) have been described and many of these are commercially available.

To facilitate the application of this approach to a wide range of target proteins, we designed our synthetic strategy to be as modular and efficient as possible, taking advantage of the power of click chemistry. Building on existing pharmacology and the growing repertoire of PCLs, PORTL compounds may be synthesized with different head groups for many other target proteins of interest. These compounds may include photoswitchable agonists, antagonists, or allosteric modulators. The ability to change linker lengths may aid in the engineering of other photoswitchable proteins. Relative to the challenge of finding optimal cysteine residues for maleimide-based photoswitch conjugation with first-generation PTLs, the PORTL system will greatly facilitate the design and implementation of new photoswitchable proteins. In addition, the PORTL system with the SNAP tag will enable the optical control of intracellular proteins because, unlike maleimide, the benzylguanine-labeling motif is unaffected by the reducing environment of the cell.

Finally, a major breakthrough in this study that is made possible by the PORTL system is the demonstration of the ability to orthogonally optically manipulate SNAG-mGluR2 and the maleimide-based LiGluR in the same cell. The ability to separately label and manipulate multiple receptor populations may be especially useful for probing crosstalk between proteins at the molecular, cellular, or circuit level. In the future, combination of SNAP-tethered photoswitches with PORTL compounds targeting the orthogonal SNAP-variant CLIP (Gautier et al., 2008) or the unrelated Halo tag (Los et al., 2008) may greatly expand the ability to optically control multiple receptor populations independently in the same preparation. Tuning of the spectral properties of the azobenzene photoswitch will further facilitate the ability to complex multiple tools within the same preparation. Overall, the PORTL approach brings us closer toward the overarching goal of obtaining the ability to individually and precisely photo-activate or inhibit the fundamental signaling molecules of the brain in concert in behaving animals.

The following examples and drawings illustrate the present invention without, however, limiting the same thereto.

### BRIEF DESCRIPTION OF THE DRAWINGS

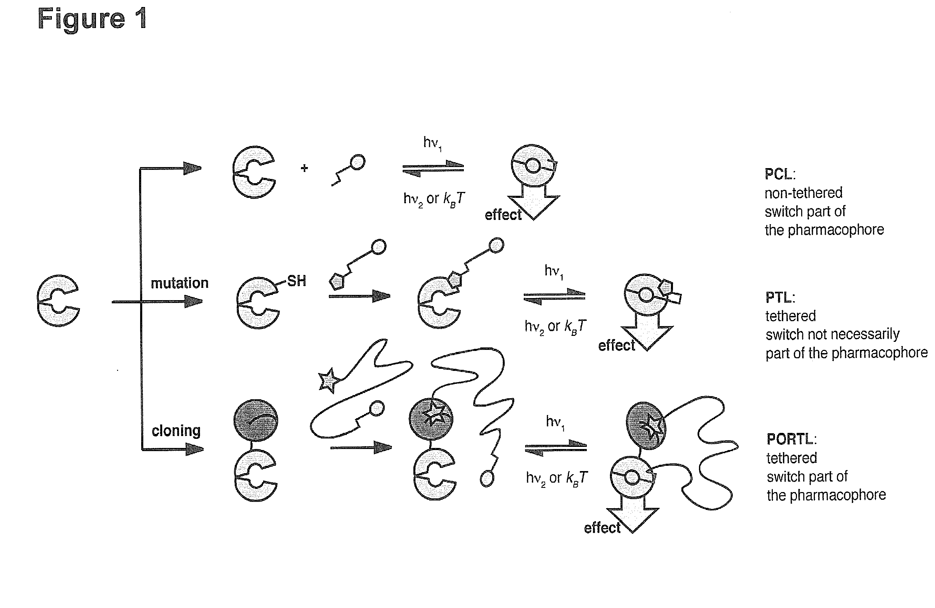

**Figure 1****:** *The PORTL concept.*
   A photochromic ligand (PCL) is freely diffusible and the switch is part of the pharmacophore (top). This is not necessarily the case in a photoswitchable tethered ligand (PTL) (middle). The photoswitchable orthogonal remotely tethered ligand approach (PORTL, bottom) combines the switch as part of the pharmacophore with a long, flexible tether that allows for anchoring on a remote site.

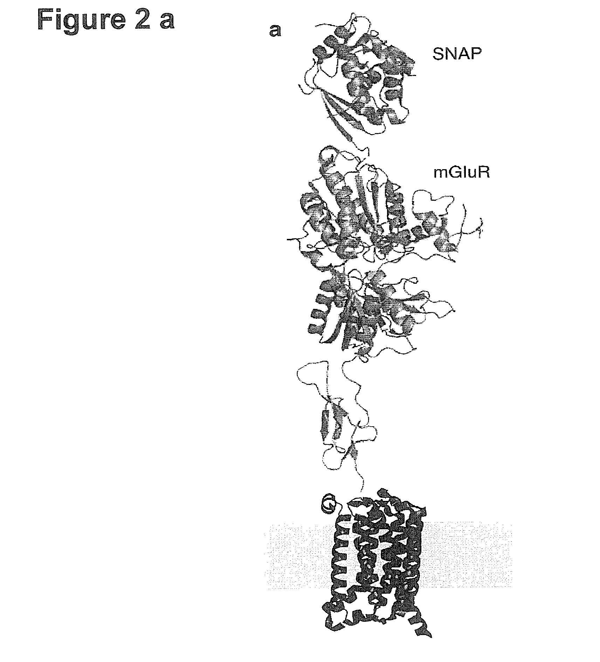

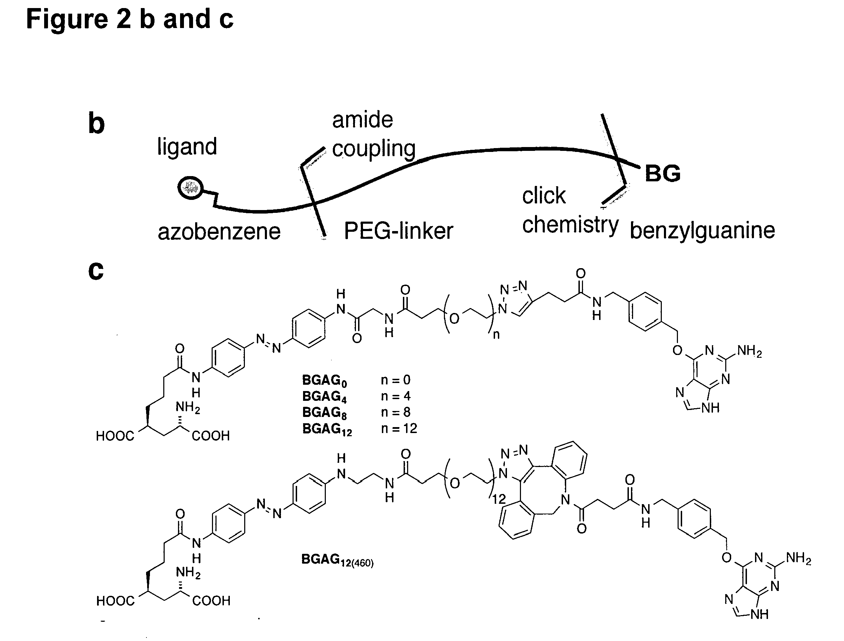

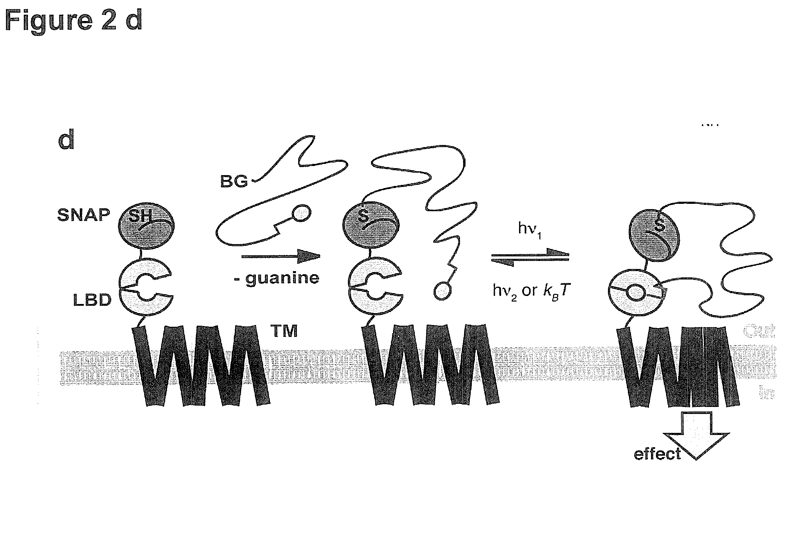

**Figure 2****:** *Concept and Design of PORTL compounds for SNAP-tag conjugation.*
   a) Model of a SNAP-mGluR subunit showing the relative dimensions of the domains (SNAP pdb: 3kzy, mGluR3-LBD pdb: 2e4u, mGluR5-7TM pdb: 4009). The mGluR extracellular domains are shown in gray and the transmembrane domains are shown in black while the SNAP tag is shown in green.
   b) Schematic design of a photoswitchable orthogonal remotely tethered ligand (PORTL) using amide coupling and click chemistry to ensure synthetic modularity.
   c) PORTL consisting of a ligand connected to an azobenzene a flexible linker (PEG-chain) of various length and a benzylguanine (**BG**). Only one regioisomer is shown in **BGAG**_{**12**(460)}. Depending on the substitution pattern on the azobenzene the switching wavelength can be tuned.
   d) Schematic showing the logic of PORTL-mediated reversible activation and deactivation of a target receptor with two orthogonal wavelengths of light.

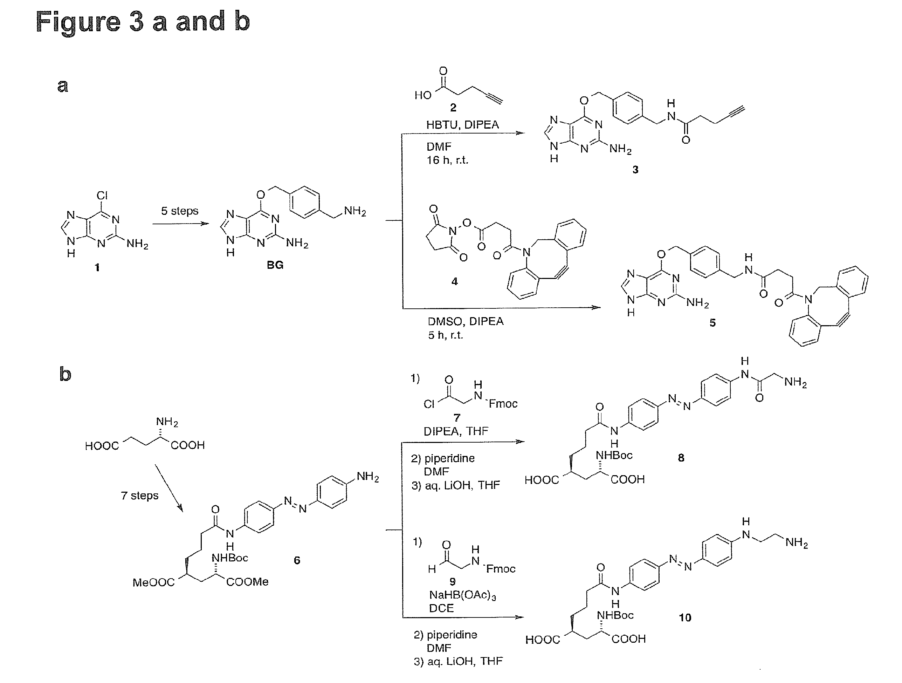

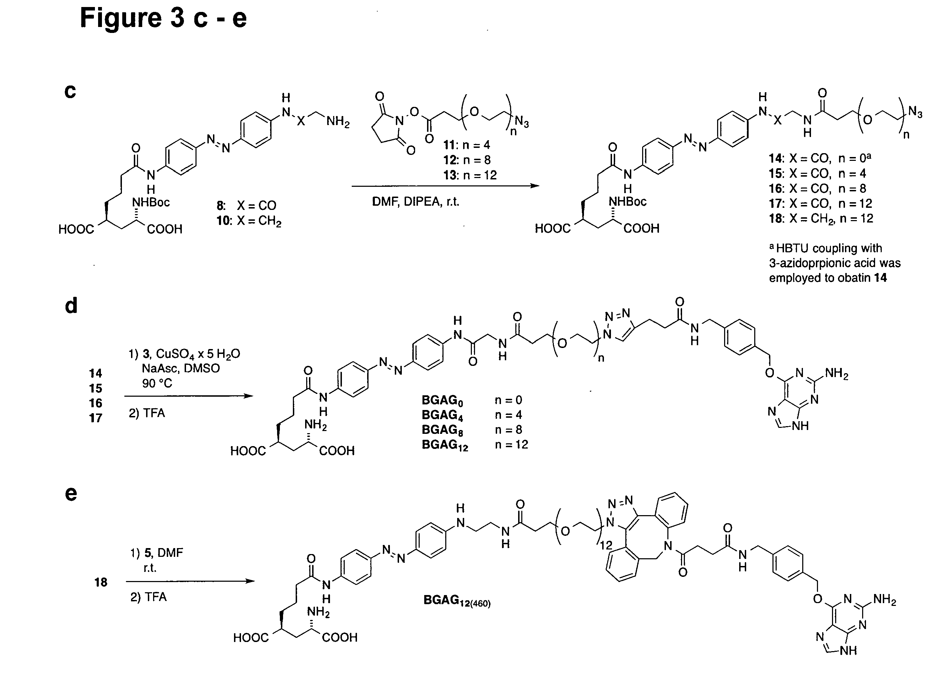

**Figure 3****:** *Synthesis of BGAGs.*
   a) Synthesis of BG-alkynes **3** and **5** for click chemistry.
   b) Diversification of previously described **6** (Levitz et al., 2013) to give blue azobenzene glutamate **8** and red-shifted azobenzene glutamate **10**.
   c) PEG-chain installation.
   d) Cu(I)-catalyzed alkyne azide click to obtain **BGAGs.**
   e) Strain promoted alkyne azide click to obtain **BGAG**_{**12**(460)}.

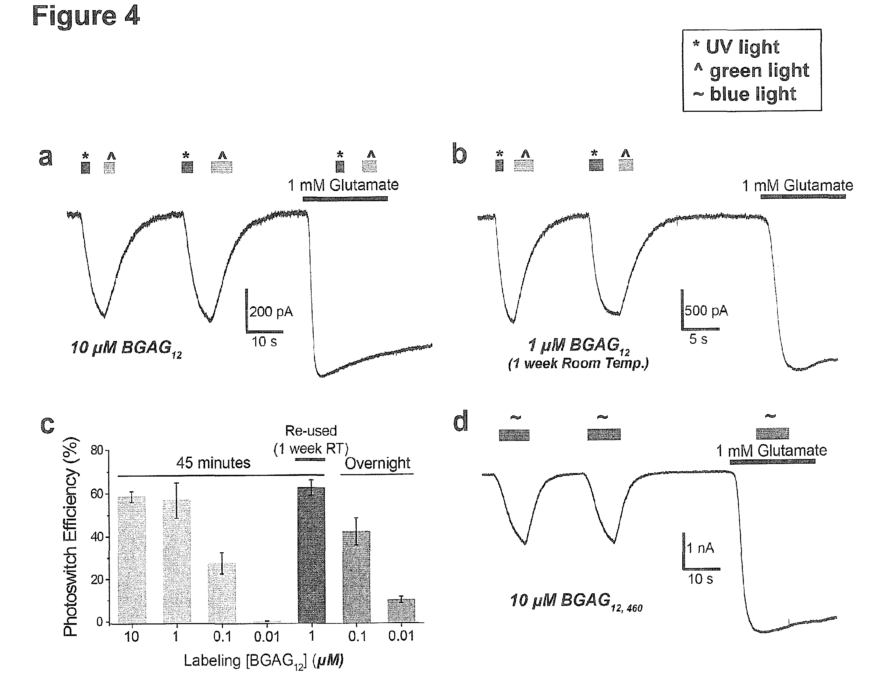

**Figure 4****:** *Optical control of SNAG-mGluR2 in HEK 293T cells co-expressing SNAP-mGluR2 and GIRK.*
   a) Representative patch-clamp trace demonstrating the reversible optical control of SNAG-mGluR2 (SNAP-mGluR2 + BGAG₁₂₍₄₆₀₎). Photoactivation is achieved with a brief pulse of UV light (λ = 380 nm, *) and reversed by a brief pulse of green light (λ = 500 nm, ^). Application of saturating 1 mM glutamate gives full activation and prevents further photoswitching.
   b) Representative trace showing photoactivation of SNAG-mGluR2 using 1 µM **BGAG₁₂** after it was incubated for 1 week in aqueous buffer.
   c) Summary of the efficiency of photoactivation of SNAG-mGluR2 (compared to 1 mM glutamate) following different **BGAG₁₂** labeling conditions. Error bars represent SEM.
   d) Representative trace showing photoactivation of SNAG₄₆₀-mGluR2 (SNAP-mGluR2 + **BGAG**_{**12**(460)}) with blue light (λ = 445 nm). Relaxation occurs spontaneously in the dark.

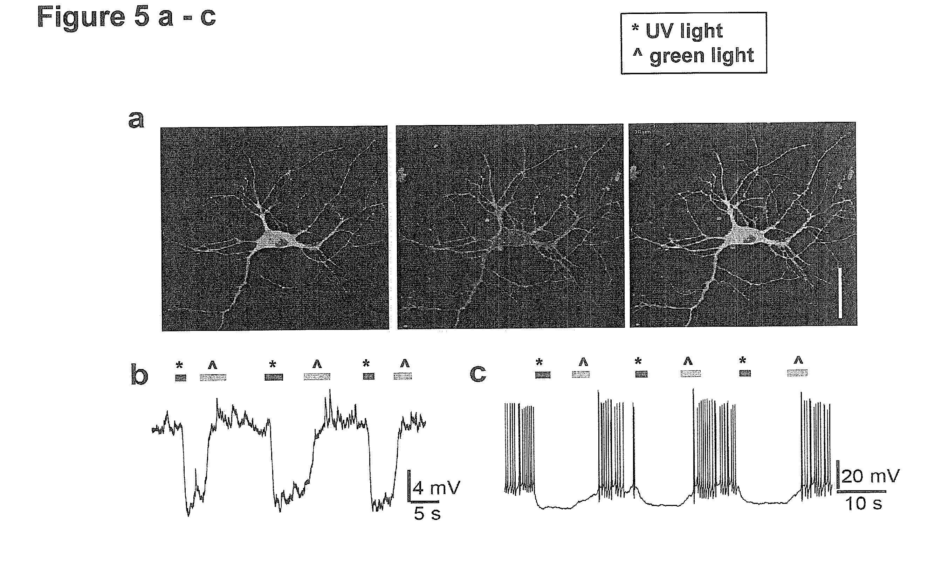

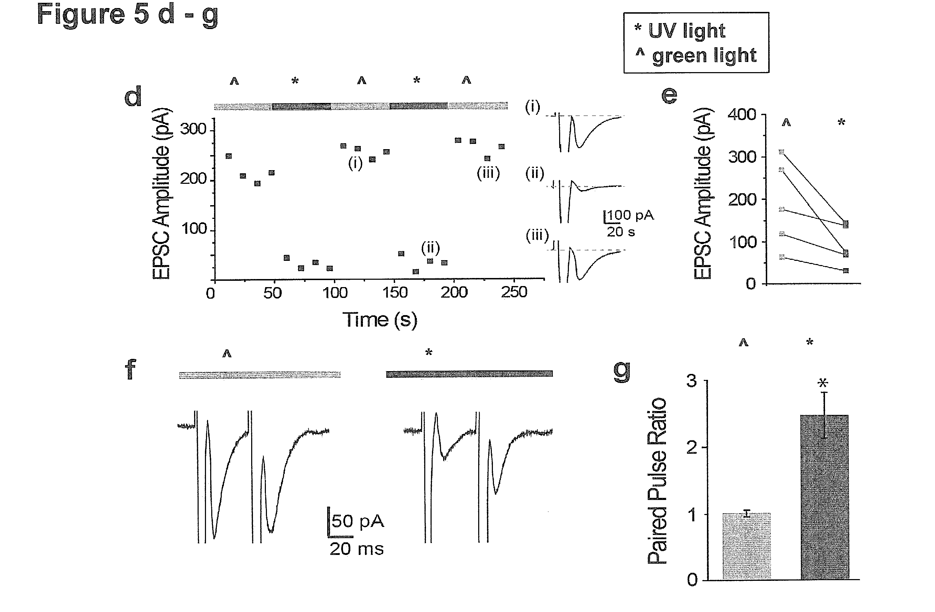

**Figure 5****:** *Optical control of SNAG-mGluR2 in hippocampal neurons.*
   a) Representative confocal images showing the expression of SNAP-mGluR2-GFP (left) and its labeling with BG-Alexa-647 (middle) in hippocampal neurons. In the merge (right) of the two images it is clear that dye labeling occurs on the surface of the neuron only.
   **b-c**) Representative recording showing SNAG-mGluR2 mediated hyperpolarization (b) and silencing (c) of hippocampal neurons in whole cell patch-clamp recordings. Violet bars (*) indicate 380 nm illumination and green bars (^) indicate 500 nm illumination.
   d) Time course of autaptic EPSC amplitude for a representative neuron showing rapid, reversible inhibition of synaptic transmission by SNAG-mGluR2. (i), (ii), and (iii) show individual traces associated with data points.
   e) Summary of SNAG-mGluR2 mediated optical synaptic inhibition by 380 nm light in all cells tested.
   f) Representative recording showing an increase in paired pulse ratio in response to SNAG-mGluR2 activation using an interstimulus interval of 50 ms. g) Summary of paired pulse ratio in 500 nm (^) or 380 nm (*) for the same cell as in f).

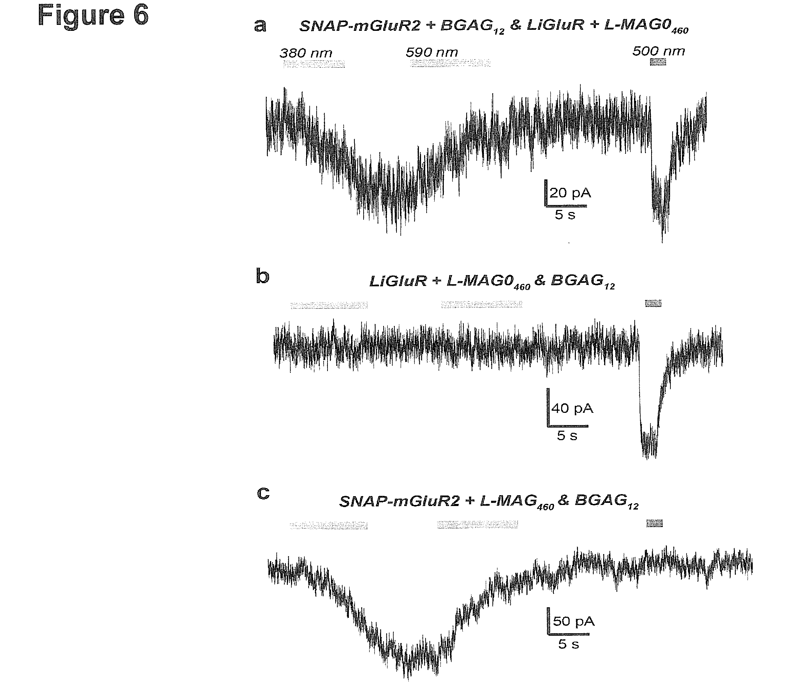

**Figure 6****:** *Dual optical control of SNAG-nzGluR2 and LiGluR in HEK 293T cells via orthogonal labeling of **BGAG₁₂** and MAG₄₆₀.*
   **a-c**) Representative traces showing the responses to dim 380 nm light (<.01 mW/mm²; purple bars), 590 nm light (~1 mW/mm²; yellow bars), and 500 nm light (~1 mW/mm²; green bars) in cells treated with **BGAG₁₂** and L-MAG0₄₆₀. Cells expressing both SNAP-mGluR2 and LiGluR show a slow SNAG-mGluR2-mediated response to 380 nm light that is reversed by 590 nm light and a fast LiGluR-mediated response to 500 nm light (a). In the absence of SNAP-mGluR2, the slow response to 380 nm is not seen (b) and in the absence of LiGluR, the fast response to 500 nm is not seen (c), conforming the origins of each current.
**Figure 7****:**
   HPLC traces of the **BGAG** library demonstrating its purity.

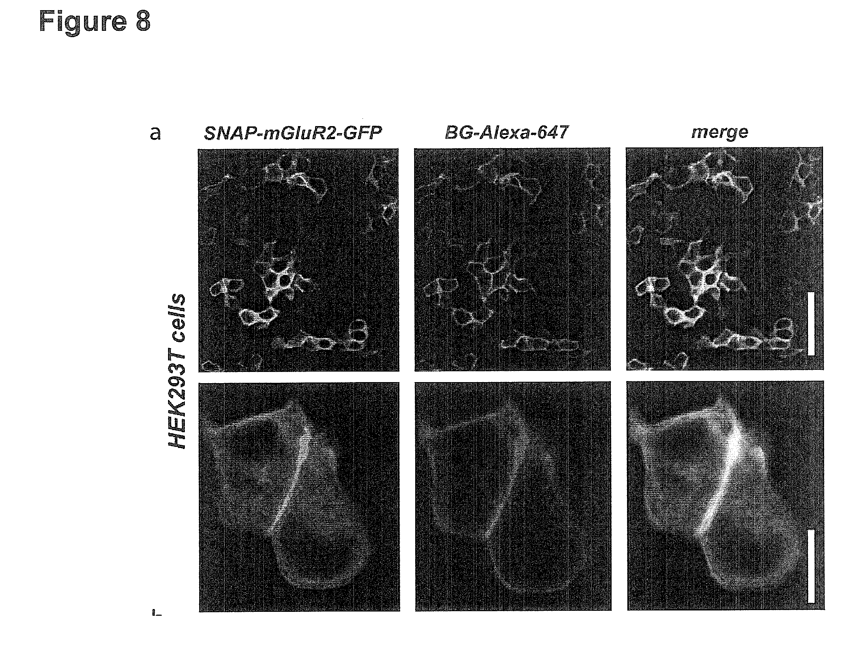

**Figure 8****:** *Expression and fluorophore labeling with benzylguanine-Alexa-647 (BG-Alexa-647) of SNAP- mGluR2-GFP in HEK293T cells.*
   First column: GFP fluorescence. Second column: BG-Alexa-647 fluorescence. Third column: merge. Scale bars represents 50 µM (top) and 10 µM (bottom).
**Figure 9****:**
   SDS page after *in vitro* SNAP-tag labeling (New England Biolabs, #P9312S) with **BGAG₁₂** and *D*-MAG. Reductive (dithiothreitol, DTT), oxidative (oxidized glutathione, GSSG) or neutral conditions (no additive) were employed according to the manufacturer's instructions. **BGAG₁₂** reacts with SNAP-tag under each condition, while *D*-MAG reacts only under oxidative and neutral conditions (although slower as indicated by the unlabeled SNAP-tag band) and is unreactive towards the SNAP-tag under reductive conditions.

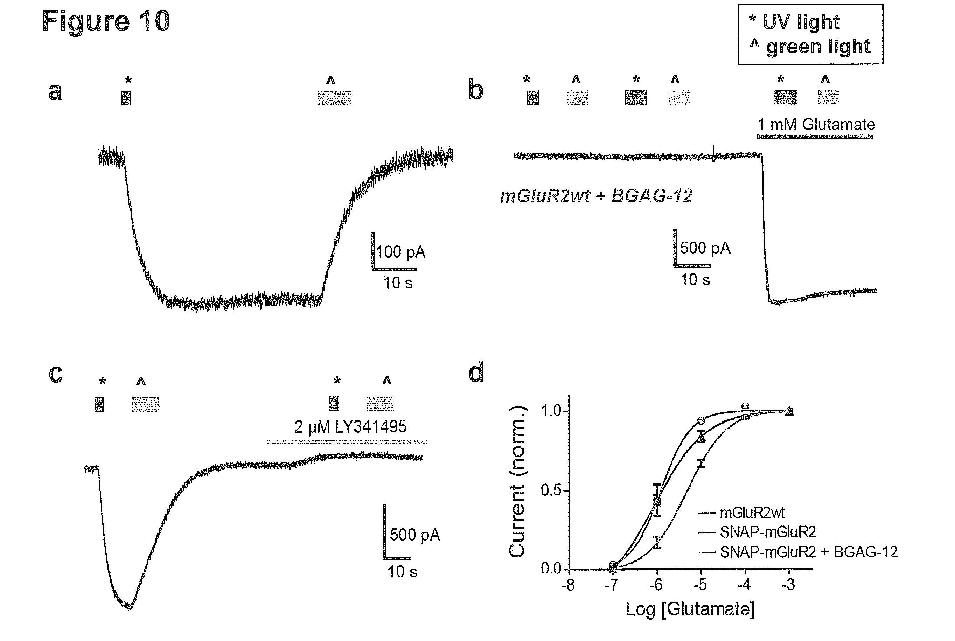

**Figure 10****:** *Further characterization of SNAG-mGluR2.*
   a) Representative patch-clamp recording demonstrates reversible, bistable optical control of SNAP-mGluR2 with **BGAG₁₂** (*i.e*. SNAG-mGluR2). SNAG-mGluR2 is activated by a brief pulse of UV light (λ = 380 nm, *) and deactivated with a brief pulse of green light (λ = 500 nm, ^). Black bar represents no light.
   b) When wild type mGluR2 (mGluR2wt) is incubated with **BGAG₁₂**, no photoresponse is seen after wash-out but application of 1 mM glutamate still produces a large inward current.
   c) Photoactivation of SNAG-mGluR2 is fully blocked by the competitive mGluR2 antagonist LY341495.
   d) Glutamate concentration-response curves for mGluR2wt and SNAP-mGluR2 with or without **BGAG₁₂** labeling. Glutamate titration was performed in the dark for all conditions.

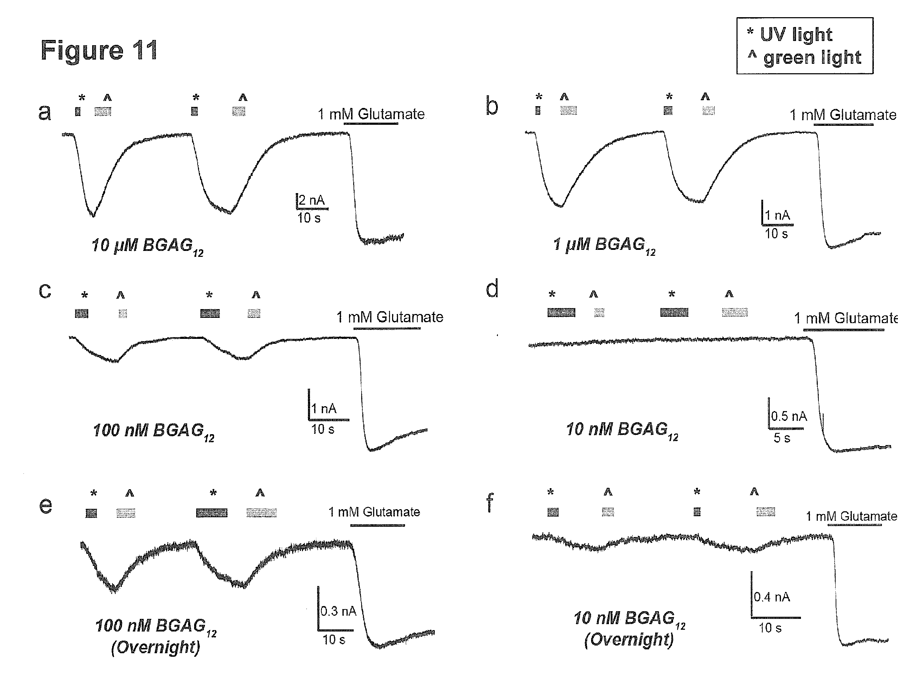

**Figure 11****:** *Summary of labeling conditions for **BGAG₁₂** on SNAP-mGluR2 in HEK293T cells.*
   **a-d**) Representative traces showing photoactivation of SNAG-mGluR2 following incubation with **BGAG₁₂** for 45 minutes in standard extracellular solution at various concentrations. (*) Bars indicate 380 nm light and (^) bars indicate 500 nm light.
   **e-f**) Representative trace showing photoactivation of SNAG- mGluR2 following overnight labeling with 100 nM (**e**) or 10 nM (**f**) **BGAG₁₂**.

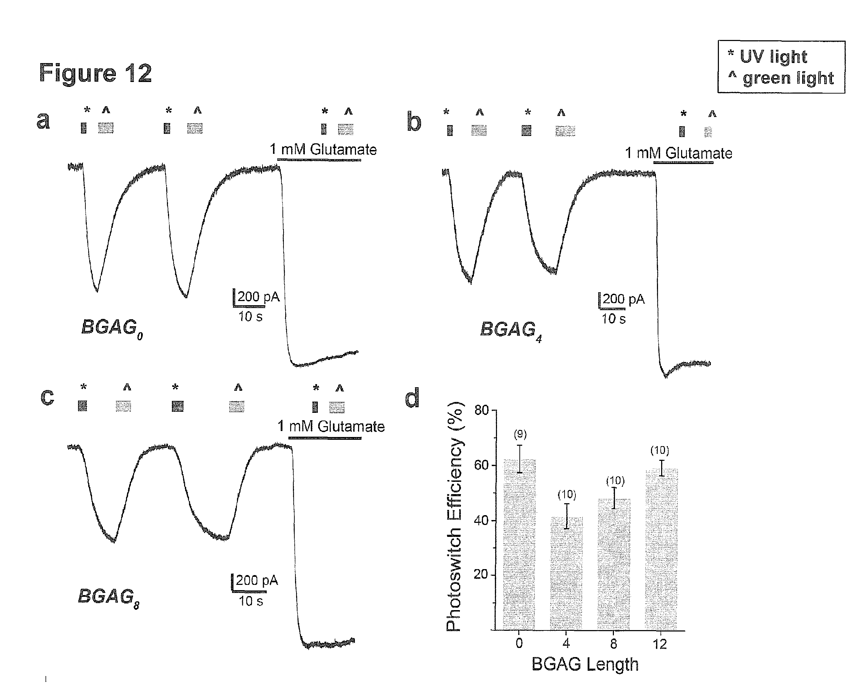

**Figure 12****:** *Optical control of SNAG-mGluR2 with variable length BGAGs in HEK293T cells.*
   **a-c**) Representative patch-clamp recording demonstrates the reversible optical control of SNAG-mGluR2 with either **BGAG₀** (**a**), **BGAG₄** (**b**), or **BGAG₈** (**c**). SNAG-mGluR2 is activated with a brief pulse of UV light (λ = 390 nm, *) and deactivated with a brief pulse of green light (λ = 500 nm, ^). Application of saturating 1 mM glutamate gives full activation and prevents further photoactivation in all cases.
   d) Summary of photoswitch efficiency relative to 1 mM glutamate for all BGAG variants. Error bars represent SEM; the numbers of cells tested are in parentheses.
**Figure 13****:** *BG-Alexa-647 labeling controls for hippocampal neurons.*
   **a-b**) Representative images showing BG-Alexa-647 labeling of GFP-expressing neurons in the absence (**a**) or presence (**b**) ofSNAP-mGluR2 coexpression.
**Figure 14****:** *SNAG-mGluR2 mediated optical modulation of short term plasticity.*
   Summary of response to high frequency (20 Hz) stimulation of a SNAG-mGluR2-expressing neuron in the presence of 380 nm (UV light) or 500 nm (green light) illumination. EPSC amplitude is normalized to the amplitude of the first pulse within the train.
**Figure 15****:** *Optical control of SNAG-mGluR2 with AGs in HEK 293T cells.*
   a) Summary of photoswitch efficiency relative to 1 mM glutamate for AG₁₂ and AG_{12,460} either treated as **BGAG₁₂** (incubated for 1 h (10µM) then washed) or with the photoswitch present (washed in (100 µM)). Error bars represent SEM; the numbers of cells tested are in parentheses.
   b) Representative patch-clamp recording demonstrates the reversible optical control of SNAG-mGluR2 with AG₁₂. SNAG-mGluR2 is activated with a UV light (λ = 380 nm, *) and deactivated with blue light (λ = 440 nm, ~). Application of saturating 1 mM glutamate gives full activation and prevents further photoactivation in all cases.

### EXAMPLES

### Example 1 Methods

### 1.1 Materials and Methods

Solvents for chromatography and reactions were purchased HPLC grade (except DMF was purchased from Acros, 99.8 %, extra dry over molecular sieves) or distilled over an appropriate drying reagent prior to use. If necessary, solvents were degassed either by freeze-pump-thaw or by bubbling N₂ through the vigorously stirred solution for several minutes. Unless otherwise stated, all other reagents were used without further purification from commercial sources.

Flash column chromatography was carried out on silica gel 60 (0.040-0.063 mm) purchased from Merck. Reactions and chromatography fractions were monitored by thin layer chromatography (TLC) on Merck silica gel 60 F254 glass plates. The plates were visualized under UV light at 254 nm.

NMR spectra were recorded in deuterated solvents on a BRUKER Avance III HD 400 (equipped with a CryoProbe™) instruments and calibrated to residual solvent peaks (¹H/¹³C in ppm): DMSO-d₆ (2.50/39.52), Me₃OD-d₄ (3.31/49.00). Multiplicities are abbreviated as follows: s = singlet, d = doublet, t = triplet, q = quartet, br = broad, m = multiplet. Spectra are reported based on appearance, not on theoretical multiplicities derived from structural information.

High-resolution electrospray (ESI) mass spectra were obtained on a Varian MAT 711 MS instrument operating in either positive or negative ionization modes.

LC-MS was performed on an Agilent 1260 Infinity HPLC System, MS-Agilent 1100 Series, Type: 1946D, Model: SL, equipped with a Agilent Zorbax Eclipse Plus C18 (100 x 4.6 mm, particle size 3.5 micron) RP column with a constant flow-rate of 2 mL/min, if not stated otherwise. Retention times (*t_{R}*) are given in minutes (min).

HPLC was performed on a Varian Prep Star HPLC System, Model SD-1 equipped with Varian Dynamax columns (RP-Analytical: Microsorb 60 C18, 250 x 4.6 mm, particle size 8 µm; RP-SemiPrep: Microsorb 60 C18, 250 x 21.4 mm, particle size 8 µm; RP-Prep: Microsorb 60 C18, 250 x 41.4 mm, particle size 8 µm). Prior to injection, samples were filtered through a syringe filter (Chromafil Xtra GF100/25, pore size 1 µm).

### 1.2 Chemical Synthesis of Photoswitches

### A. General procedure for attachement of the Azide-functionalized PEG-linker

In a schlenk flash, **8** or *N*-Boc-*D*-redAG0 **10** (1.1 equiv.), Azido-(PEG)ₙ-NHS-ester (Baseclick, n = 4 (**11**, #BCL-001), n = 8 (**12**, #BCL-032), n = 12 (**13**, #BCL-033); 1.0 equiv.) and DIPEA (2.0 equiv.) were added to degassed, anhydrous DMF (0.5 mL) under N₂-atmosphere. The reaction mixture was stirred at r.t. while reaction progress was monitored by LCMS. Upon completion, the crude reaction mixture was purified by C18 reverse phase (RP) flash column chromatography (100/0 → 60/40 = 1 mM HCl/MeCN) or by RP-HPLC (MeCN/H₂O/formic acid = 10/90/0.1 → 80/20/0.1 over 42 min). The product containing fractions were combined, concentrated *in vacuo* and freeze-dried to obtain the desired product.

### B. General procedure for click reaction

In a schlenk flask, the reaction mixture was prepared with the corresponding azide (1.0 equiv.) and BG-alkyne (**SI2**.**1**) (1.1 eq) in degassed DMSO (2-4 mL) under N₂-atmosphere. Stock solutions of sodium ascorbate (NaAsc, 131 mM) and copper-(II)-sulfate pentahydrate (105 mM) were prepared separately in degassed water. 50 µL of each stock solution were mixed under N₂ to preform the catalytically active Cu^{I} species and quickly transferred to the reaction mixture. The resulting reaction mixture was heated to 90 C and reaction progress was monitored by LCMS. Upon completion, the crude product was purified by RP flash column chromatography (100/0 → 60/40 = 1 mM HCl/MeCN) or RP-HPLC (MeCN/H₂O/formic acid = 10/90/0.1 → 80/20/0.1 over 42 min). The product containing fractions were combined, concentrated *in vacuo* and freeze-dried to obtain the desired product.

### C. General procedure for Boc-deprotection:

In a falcon tube, neat TFA (250 µL) was added to the Boc-protected molecule and stirred at r.t. for 10 min. Diethylether (50 mL) was added and the resulting suspension was centrifuged (4000 rpm, 20 min, 4 C). The supernatant was discarded, the solid was washed again with diethylether and finally dried under high vacuum to obtain the desired product.

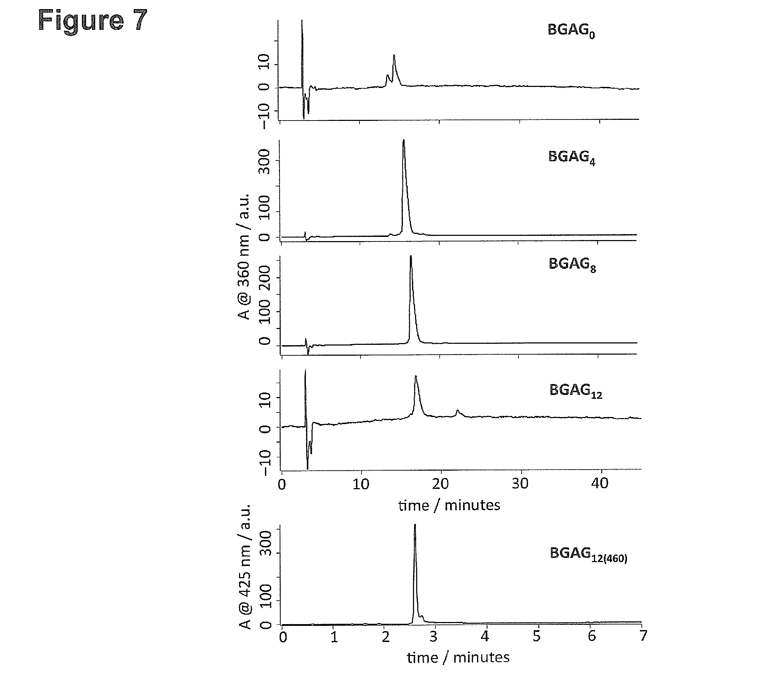

Details on the chemical synthesis of BGAGs and their precursors and characterization data can be found in the following. The purity of all **BGAG**s was assessed by 1H-NMR, HR-MS and HPLC (see Figure 7).

### N-(4-(((2-Amino-9H-purin-6-yl)oxy)methyl)benzyl)pent-4-ynamide (3)

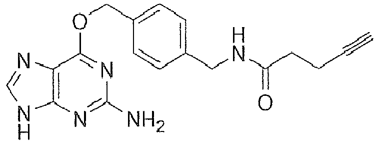

6-((4-(Aminomethyl)benzyl)oxy)-9*H*-purin-2-amine **(BG)** (Keppler et al., 2003) (382 mg, 1.41 mmol, 1.2 equiv.), 4-pentynoic acid (**2**) (115 mg, 1.17 mmol, 1.0 equiv.), HBTU (489 mg, 1.29 mmol, 1.1 equiv.), DIPEA (302 mg, 2.34 mmol, 409 µL, 2.0 equiv.) were dissolved in DMF (5 mL). Reaction progress was monitored by LCMS and after completion, the crude reaction mixture was subjected to RP-HPLC (MeCN/H₂O/FA = 10/90/0.1 → 80/20/0.1 over 40 min). The product containing fractions were combined, concentrated *in vacuo* and dried under high vacuum to obtain the product BG-alkyne (**3**) (178 mg, 0.508 mmol) as a white powder in 43% yield.

NMR spectroscopy revealed two rotamers, proven by heating the NMR sample to 50 □C and merging of the spectroscopic signals (data not shown). Peaks are reported for the major rotamer.

**¹H NMR** (400 MHz, DMSO-d₆): δ [ppm] = 8.45-8.34 (m, 1H), 8.13 (s, 1H), 8.10 (s, 1H), 7.47 (d, *J*= 8.4 Hz, 2H), 7.29 (d, *J* = 8.4 Hz, 2H), 6.59 (br s, 2H), 5.48 (s, 2H), 4.32-4.21 (m, 2H), 2.78 (m, 1H), 2.43-2.28 (m, 4H).

**¹³C NMR** (101 MHz, DMSO-d₆): δ [ppm] = 170.3, 159.2, 159.1, 155.0, 141.0, 139.5, 134.7, 128.7, 127.3, 126.4, 83.8, 71.4, 67.2, 41.9, 34.2, 14.3.

**HRMS (ESI):** calc. for C₁₈H₁₉N₆O₂+ (M+H)⁺: 351.1564, found: 351.1562.

**UV**/**Vis** (LCMS): λₘₐₓ₁ = 196 mn, λₘₐₓ₂ = 212 nm, λₘₐₓ₃ = 287 nm.

***t*_{R}** (LCMS; MeCN/H₂O/formic acid = 10/90/0.1 → 90/10/0.1 over 7 min) = 1.955 min.

### N-(4-(((2-Amino-9H-purin-6-yl)oxy)methyl)benzyl)-4-(11,12-dehydrodibenzo[b,f]azocin-5(6H)-yl)-4-oxobutanamide (5)

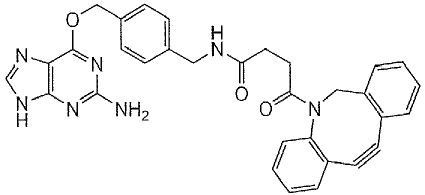

6-((4-(Aminometbyl)benzyl)oxy)-9*H*-purin-2-amine (**BG**) (Keppler et al., 2003) (6.7 mg, 24.9 µmol, 1.0 equiv.), DBCO-NHS-ester (Jena Bioscience, CLK-A133-25, **4**) (10.0 mg, 24.9 µmol, 1.0 equiv.), DIPEA (6.4 mg, 49.8 µmol, 8.7 µL, 2.0 equiv.) were dissolved in DMSO (0.5 mL). Reaction progress was monitored by LCMS and after completion the crude reaction mixture was subjected to RP-HPLC (McCN/H₂O/FA = 5/95/0.1 → 80/20/0.1 over 40 min). The product containing fractions were combined, concentrated *in vacuo* and dried under high vacuum to obtain **5** (1.0 mg, 1.79 µmol) in 7% yield. The low yield can be attributed to residual water in the used DMSO as the corresponding DBCO-acid was obtained as the main product.

**HRMS (ESI):** calc. for C₃₂H₂₈N₇O₃⁺ [M+H]⁺: 558.2248, found: 558.2254.

**UV**/**Vis** (LCMS): λₘₐₓ = 290 nm.

*t*_{R} (LCMS; MeCN/H₂O/formic acid = 10/90/0.1 → 90/10/0.1 over 7 min) = 3.118 min.

### (2S,4S)-2-(4-((4-((E)-(4-(2-Aminoacetamido)pbenyl)diazenyl)phenyl)amino)-4-oxobutyl)-4-((tert-butoxycarbonyl)amino)pentanedioic acid (6)

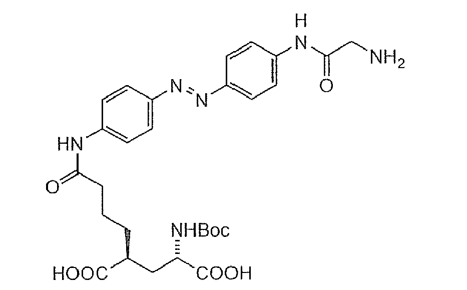

**6** was prepared according to a literature procedure and analytical data matched the one reported (see Levitz et al., 2013).

**¹H-NMR** (400 MHz, _{DMSO}-d₆) δ [ppm] = 10.29 (s, 1H), 7.93-7.75 (m, 9H), 6.60 (d, *J* = 7.8 Hz, 1H), 3.90 (q, *J* = 8.1 Hz, 1H), 3.78 (s, 2H), 2.44-2.25 (m, 3H), 1.88-1.42 (m, 6H), 1.36 (s, 9H).

**¹³C NMR** (101 MHz, DMSO-d₆) δ [ppm] = 176.3, 174.0, 171.5, 165.9, 155.1, 148.0, 147.4, 142.2, 140.9, 123.5, 123.4, 119.4, 119.2, 77.9, 55.0, 52.0, 41.6, 40.1, 36.5, 30.9, 28.2, 23.0. **HRMS** (**ESI**)**:** calc. for C₂₈H₃₇N₆O₈⁺ [M+H]⁺: 585.2667, found: 585.2673.

**UV/VIS** (LCMS): λₘₐₓ → π□) = 368 nm.

***t*_{R}** (LCMS, MeCN/H₂O/formic acid = 90/10/0.1 → 10/90/0.1 over 7 min) = 2.418 min.

### (2S,4S)-2-(4-((4-((E)-(4-(2-(3-azidopropanamido)acetamido)phenyl)diazenyl)phenyl)-amino)-4-oxobutyl)-4-((tert-butoxycarlbonyl)amino)pentanedioic acid (14)

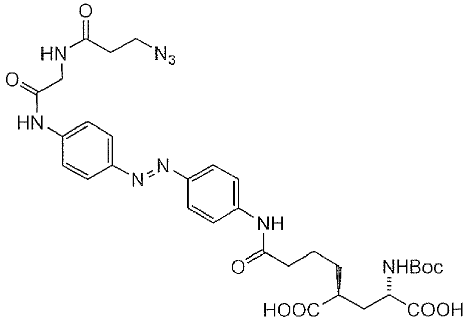

A schlenk flask was charged with **8** (10 mg, 17 µmol, 1.0 equiv.), HBTU (7.2 mg, 19 µmol, 1.1 equiv.) and 3-azidopropionic acid (Di Antonio et al., 2012) (3.0 mg, 16 µmol, 1.5 equiv.) under a N₂-atmosphere. Degassed, anhydrous DMF (1 mL) was added and the reaction mixture was cooled to 0 °C before DIPEA (6.0 µL, 34 µmol, 2.0 equiv.) was added dropwise. The reaction mixture was allowed to warm to r.t. and reaction progress was monitored by LCMS. Upon completion, the crude reaction mixture was purified by RP-HPLC (10/90/0.1 →80/20/0.1 = MeCN/H₂O/formic acid over 45 min). The product containing fractions were combined, concentrated *in vacuo* and freeze-dried to obtain 10 mg (15 µmol, 88%) of **14** as a yellow solid.

**¹H NMR** (400 MHz, DMSO-d₆) δ [ppm] = 10.34 (s, 1H), 10.25 (s, 1H), 8.43 (t, *J=* 5.8 Hz, 1H), 7.98-7.71 (m, 8H), 3.96 (d, *J* = 5.7 Hz, 2H), 3.89 (t, *J =* 7.3 Hz, 1H), 3.53 (t, *J=* 6.4 Hz, 2H), 2.67 (s, 1H), 2.43-2.23 (m, 4H), 1.86-1.42 (m, 6H), 1.36 (s, 9H).

**HRMS** (ESI): calc. for C₃₁H₃₈N₉O₉⁻ [M-H]⁻: 680.2798, found: 680.2798.

**t_{R}** (RP-HPLC, _{MeCN}/H₂O/formic acid = 10/90/0.1 → 80/20/0.1 over 42 min)= 26.2 min (*trans*)

### (2S,4S)-2-Amino-4-(4-((4-((E)-(4-(2-(3-(4-(3-((4-(((2-amino-9H-purin-6-yl)oxy)methyl) benzyl)amino)-3-oxopropyl)-1H-1,2,3-triazol-1-yl)propanamido)acetamido)-phenyl)diazenyl)phenyl)amino)-4-oxobutyl)pentanedioic acid, BGAG₀

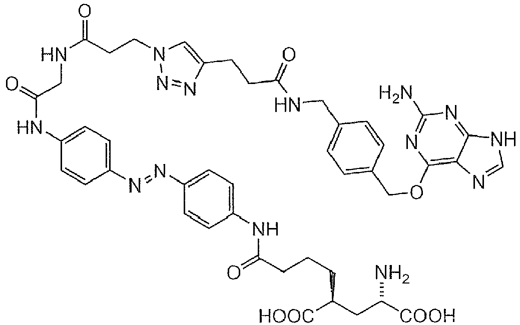

**BGAG₀** was prepared according to general procedures B and C.

### Amounts:

**3** (5.8 mg, 17 µmol, 1.1 equiv.)
**14** (10 mg, 15 µmol, 1.0 equiv.)

yield: 3.3 mg (3.5 µmol, 23% over two steps), yellow solid.

**¹H NMR** (400 MHz, DMSO-d₆) δ 10.52 (s, 1H), 10.33 (s, 1H), 10.27 (s, 1H), 8.51-8.24 (m, 3H), 7.96-7.59 (m, 8H), 7.52 (dd, *J=* 8.9, 3.1 Hz, 1 H), 7.41 (m, 2H), 7.23 (t, *J* = 9.0 Hz, 3H), 7.18-7.06 (m, 1H), 6.98 (s, 1H), 6.82 (t, *J*= 7.4 Hz, 1H), 6.29 (br s, 2H), 5.42 (br s, 2H), 4.52 (t, *J=* 7.0 Hz, 2H), 4.27 (t, *J=* 4.9 Hz, 2H), 3.94 (d, *J* = 5.7 Hz, 2H), 3.60 (s, 1H), 3.16 (d, *J =* 4.0 Hz, 1H), 2.90-2.83 (m, 2H), 2.79 (t, *J=* 6.8 Hz, 2H), 2.67 (p, *J*= 1.7 Hz, 1H), 2.41-2.31 (m, 3H), 1.88-1.33 (m, 6H).

**HRMS** (ESI): calc. for C₄₄H₄₈N₁₅O₉⁻ [M-H]⁻: 930.3765, found: 930.3770.

***t*_{R}** (RP-HPLC, MeCN/H₂O/formic acid = 10/90/0.1 → 80/20/0.1 over 42 min) = 18.4 min (*trans,* before Boc-deprotection).

**UV/VIS** (LCMS): λₘₐₓ (π → π^{□}) = 368 nm.

***t*_{R}** (LCMS, MeCN/H₂O/formic acid = 90/10/0.1 → 10/90/0.1 over 10 min, flow: 1 ml/min) = 3.960 min.

### (2S,4S)-2-(4-((4-((E)-(4-((1-Azido-15-oxo-3,6,9,12-tetraoxa-16-azaoctadecan-18-yl)amino)phenyl)diazenyl)phenyl)amino)-4-oxobutyl)-4-((tert-butoxycarbonyl)-amino)pentanedioic acid (15)

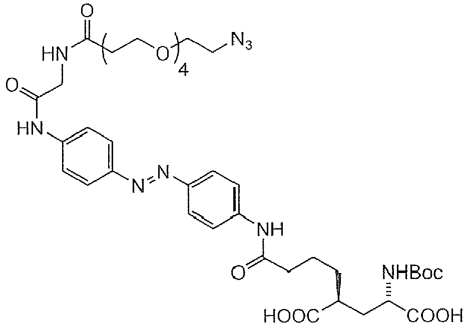

**15** was prepared according to general procedure A.

Amounts:

N₃-PEG₄-NHS-ester (#BCL-001, 8.6 mg, 22 µmol, 1.1 equiv.)
**8** (14.3 mg, 24 µmol, 1.0 equiv.)

DIPEA (7.7 µL, 44 µmol, 2.0 equiv.)

yield: 18.0 mg (21 µmol, 86%), yellow solid.

**HRMS** (ESI): calc. for C₃₉H₅₄N₉O₁₃⁻ [M-H]⁻: 856.3847, found: 856.3844.

**UV/VIS** (LCMS): λₘₐₓ (π → π^{□}) = 368 nm.

***t_{R}*** (LCMS, MeCN/H₂O/formic acid = 90/10/0.1 → 10/90/0.1 over 10 min) = 5.130 min.

**(2*S*,4*S*)-2-Amino-4-(4-((4-((*E*)-(4-(1-(4-(3-((4-(((2-amino-9*H*-purin-6-yl)oxy)methyl)-benzyl)amino)-3-oxopropyl)-1*H*-1,2,3-triazol-1-yl)-15-oxo-3,6,9,12-tetraoxa-16-aza-octadecan-18-amido)phenyl)diazenyl)phenyl)amino)-4-oxobutyl)-pentanedioic acid,**

### BGAG₄

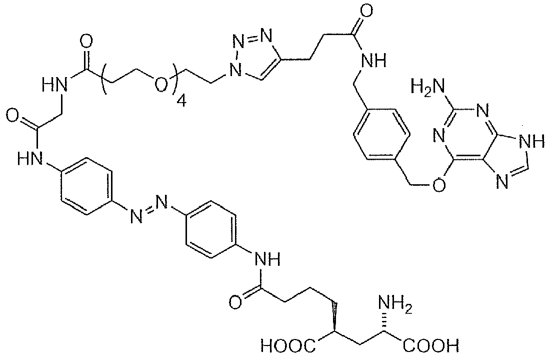

**BGAG₄** was prepared according to general procedures B and C.

### Amounts:

**3** (7.8 mg, 23 µmol, 1.1 equiv.)
**15** (18 mg, 21 µmol, 1.0 equiv)

yield: 5.1 mg (5 µmol, 24% yield over two steps).

**¹H NMR** (400 MHz, DMSO-d₆) δ [ppm] =10.58 (s, 1H), 10.33 (s, 1H), 10.30 (s, 1H), 8.37 (t, *J=* 6.2 Hz, 2H), 8.28 (t, *J*= 5.8 Hz, 2H), 7.89-7.69 (m, 12H), 7.23 (d, *J =* 7.9 Hz, 2H), 7.14 (d, *J=* 7.9 Hz, 2H), 6.28 (s, 2H), 4.44 (t, *J*= 5.1 Hz, 4H), 4.23 (d, *J=* 5.9 Hz, 2H), 3.93 (d, *J* = 5.7 Hz, 2H), 3.77 (t, *J=* 5.3 Hz, 3H), 3.62 (t, *J*= 6.5 Hz, 2H), 3.54-3.44 (m, 12H), 2.86 (t, *J* = 7.6 Hz, 2H), 2.45-2.30 (m, 5H), 1.97-1.72 (m, 3H), 1.70-1.39 (m, 6H).

**HRMS** (ESI): calc. for C₅₂H₆₆N₁₅O₁₃⁺ [M+H]⁺: ₁₁₀₈.4959,found: 1108.4943.

**UV/VIS** (LCMS): λₘₐₓ (π → π^{□}) = 368 nm.

***t_{R}*** (LCMS, MeCN/H₂O/formic acid = 90/10/0.1 → 10/90/0.1 over 10 min) = 2.927 min.

### (2S,4S)-2-(4-((4-((E)-(4-((1-Azido-27-oxo-3,6,9,12,15,18,21,24-octaoxa-28-azatriacontan-30-yl)amino)phenyl)diazenyl)-phenyl)amino)4-oxobutyl)-4-((tert-butoxycarbonyl)-amino)pentanedioic acid (16)

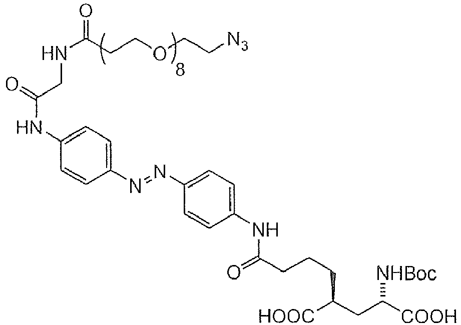

**16** was prepared according to general procedure A.

### Amounts:

N₃-PEG₈-NHS-ester (#BCL-032, 10.0 mg, 17.7 µmol, 1.0 equiv.)
**8** (11.4 mg, 19.5 µmol, 1.1 equiv.)
DIPEA (6.2 µL, 35.4 µmol, 2.0 equiv.)

yield: 18.0 mg (17 µmol, 89%), yellow solid.

**HRMS** (ESI): calc. for C₄₇H₆₉N₉O₁₇²⁻ [M-2H]²⁻: 515.7411, found: 515.7407.

**UV/VIS** (LCMS): λₘₐₓ (π → π^{□}) = 368 mn.

***t_{R}*** (LCMS, MeCN/H₂O/formic acid = 90/10/0.1 → 10/90/0.1 over 10 min) = 4.998 min.

### (2S,4S)-2-Amino-4-(4-((4-((E)-(4-(1-(4-(3-((4-(((2-amino-9H-purin-6-yl)oxy)methyl)-benzyl)amino)-3-oxopropyl)-1H-1,2,3-triazol-1-yl)-27-oxo-3,6,9,12,15,18,21,24-octa-oxa-28-azatriacontan-30-amido)phenyl)diazenyl)phenyl)amino)-4-oxobutyl)pentane-dioic acid, BGAG₈

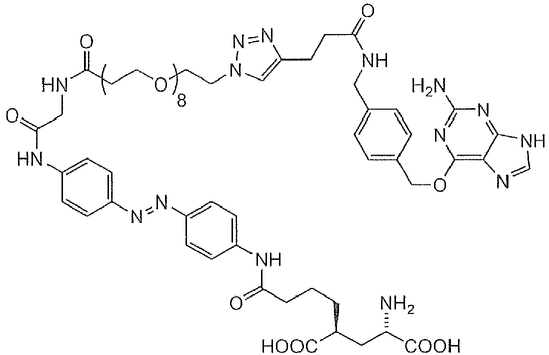

**BGAG₈** was prepared according to general procedures B and C.

### Amounts:

3 (7.1 mg, 21 µmol, 1.2 equiv.)
**16** (18 mg, 17 µmol, 1.0 equiv.)

yield: 9.6 mg (7.5 µmol, 44% yield over two steps), yellow solid.

**¹H NMR** (400 MHz, DMSO-d₆) δ 10.91 (s, 1H), 10.32 (s, 1H), 10.29 (s, 1H), 8.43-8.20 (m, 5H), 8.09 (s, 1H), 7.89-7.72 (m, 10H), 7.23 (d, *J* = 7.7 Hz, 2H), 7.14 (d, *J* = 7.7 Hz, 2H), 6.55 (s, 2H), 4.73 (s, 1H), 4.50-4.39 (m, 4H), 4.23 (d, *J=* 6.3 Hz, 2H), 3.93 (d, *J* = 5.8 Hz, 2H), 3.77 (t, *J*= 5.3 Hz, 2H), 3.62 (t, *J* = 6.6 Hz, 2H), 3.49 (d, *J=* 4.6 Hz, 28H), 2.86 (t, *J=* 7.7 Hz, 2H), 2.46-2.31 (m, 6H), 2.10-1.96 (m, 1H), 1.85 (dt, *J* = 14.2, 7.0 Hz, 1H), 1.58 (d, *J* = 6.3 Hz, 5H).

**HRMS** (ESI): calc. for C₆₀H₈₂N₁₅O₁₇⁺ [M+H]⁺: 1284.6008, found: 1284.6004.

**UV/VIS** (LCMS): λₘₐₓ (π → π^{□})= 368 mn.

*t*_{R} (LCMS, MeCN/H₂O/formic acid = 90/10/0.1 → 10/90/0.1 over 10 min) = 3.059 min.

### (2S,4S)-2-(4-((4-((E)-(4-(1-Azido-39-oxo-3,6,9,12,15,18,21,24,27,30,33,36-dodecaoxa-40-azadotetracontan-42-amido)phenyl)diazenyl)phenyl)amino)-4-oxobutyl)-4-((tert-butoxycarbonyl)amino)pentanedioic acid (17)

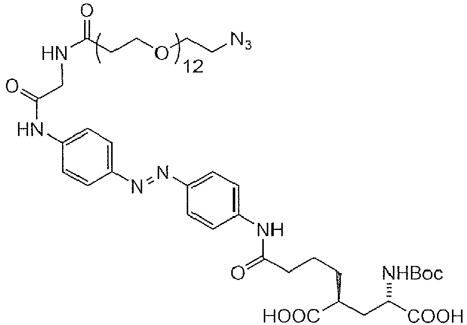

**17** was prepared according to general procedure A.

### Amounts:

N₃-PEG₁₂-NHS-Ester (Baseclick #BCL-033, 10.0 mg, 13.5 µmol, 1.0 equiv.)
**8** (8.7 mg, 14.8 µmol, 1.1 equiv.)
DIPEA (3.5 mg, 27.0 µmol, 4.7 µL, 2.0 equiv.)

yield: 13.0 mg (10.8 µmol, 80%), yellow solid.

**HRMS (ESI):** calc. for C₅₅H₈₅N₉O₂₁²⁻ [M-2H]²⁻: 603.7936, found: 603.7937. **UV/Vis** (LCMS): λₘₐₓ (π → π*) = 368 nm.

***t*_{R}** (LCMS; MeCN/H₂O/formic acid = 10/90/0.1 → 90/10/0.1 over 7 min) = 3.232 min.

### (2S,4S)-2-Amino-4-(4-((4-((E)-(4-(1-(4-(3-((4-(((2-amino-9H-purin-6-yl)oxy)methyl)benzyl)amino)-3-oxopropyl)-1H-1,2,3-triazol-1-yl)-39-oxo-3,6,9,12,15,18,21,24,27,30,33,36-dodecaoxa-40-azadotetracontan-42-amido)phenyl)diazenyl)phenyl)amino)-4-oxobutyl)pentanedioic acid, BGAG₁₂

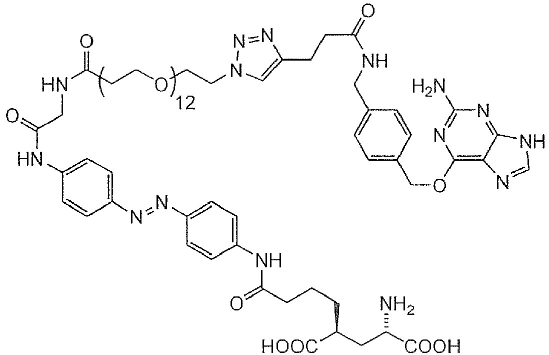

**BGAG₁₂** was prepared according to general procedures B and C.

### Amounts:

**3** (4.2 mg, 11.9 µmol, 1.2 equiv.)
**17** (12.0 mg, 9.9 µmol, 1.0 equiv.)

yield: 6.8 mg (7.06 µmol, 65% over two steps), orange solid.

**¹H NMR** (400 MHz, DMSO-d₆): δ [ppm] =10.30 (s, 1H), 10.26 (s, 1H), 8.47 (br s, 1H), 8.81 (t, *J* = 6.0 Hz, 1H), 8.36-8.16 (m, 3H), 7.93-7.68 (m, 8H), 7.48 (d, *J* = 6.0 Hz, 2H), 7.24 (d, *J* = 6.0 Hz, 2H), 6.88-6.74 (m, 1H), 5.53 (br s, 2H), 4.44 (t, *J=* 5.2 Hz, 2H), 4.27 (d, *J=* 6.0 Hz, 2H), 3.94 (d, *J* = 5.6 Hz, 2H), 3.87-3.78 (m, 1H), 3.76 (t, *J* = 5.2 Hz, 2H), 3.63 (t, *J* = 6.8 Hz, 2H), 3.56-3.41 (m, 44H), 2.86 (t, *J* = 8.0 Hz, 2H), 2.62-2.55 (m, 2H), 2.46-2.28 (m, 7H), 2.16-1.91 (m, 2H), 1.90-1.74 (m, 1H), 1.66-1.51 (m, 4H), 1.48-1.28 (m, 2H). **HRMS (ESI):** calc. for C₆₈H₉₉N₁₅O₂₁²⁺[M+2H]²⁺: 730.8565, found: 730.8564.

**UV/Vis** (LCMS): λₘₐₓ (π → π*) = 369 nm.

***t*_{R}** (LCMS; MeCN/H₂O/formic acid = 10/90/0.1 → 90/10/0.1 over 7 min) = 2.463 min.

### (2S,4S)-2-(4-((4-((E)-(4-((2-aminoethyl)amino)_{phenyl})diazenyl)phenyl)amino)-4-oxobutyl)-4-((tert-butoxycarbonyl)amino)pentanedioic acid (10)

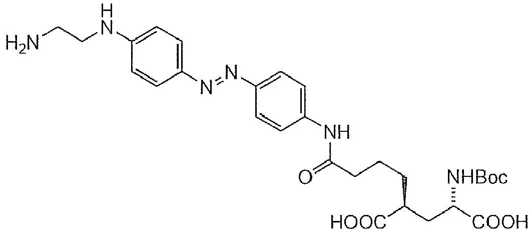

Dimethyl (2S,4S)-2-(4-((4-((E)-(4-aminophenyl)diazenyl)phenyl)amino)-4-oxobutyl)-4-((tert-butoxycarbonyl)amino)pentanedioate (Levitz et al., 2013) (200 mg, 0.360 mmol, 1.0 equiv.) and Fmoc-2-aminoacetaldehyde (Chen and Aduda, 2007) (101 mg, 0.360 mmol, 1.0 equiv.) were dissolved in DCE (10 mL) and one drop of acetic acid was added. Sodium triacetoxyborohydride (270 mg, 1.28 mmol, 3.6 equiv.) was added portionwise and the reaction mixture was stirred at r.t. for 4.5 h. The reaction mixture was quenched and subsequently washed with sat. aq. NaHCO₃ (3x) before the organic layer was concentrated *in vacuo.* The residue was redissolved in DMF (47.5 mL), piperidine (2.5 mL) was added and the deprotecting reaction was stirred overnight. The reaction mixture was concentrated *in vacuo*, the residue was redissolved in EtOAc, washed with sat. aq. NaHCO₃ (2x) and brine (sat.) before the organic layer was dried over MgSO₄. The solvent was removed *in vacuo* before purification by flash column chromatography (90/10/1 = DCM/MeOH/TEA) followed by RP column chromatography (100/0 → 60/40 = 1mM HCl/MeCN). The product containing fractions were combined, concentrated *in vacuo* and freeze-dried to obtain the intermediate bis-methylester dimethyl (2*S*,4*S*)-2-(4-((4-((*E*)-(4-((2-aminoethyl)amino)phenyl)diazenyl)phenyl)amino)-4-oxobutyl)-4-((*tert-*butoxycarbonyl)amino) pentane-dioate as its red-orange HCl salt (84 mg, 0.13 mmol) in 36% yield over 2 steps.

**¹H-NMR** (400 MHz, MeOD-d₄) δ [ppm] = 7.82-7.66 (m, 6H), 6.80 (d, *J* = 8.9 Hz, 2H), 4.16 (dd, *J* = 8.4, 6.2 Hz, 1H), 3.71-3.65 (m, 6H), 3.55 (t, *J* = 6.1 Hz, 2H), 3.18 (t, *J* = 6.8 Hz, 2H), 2.52 (p, *J* = 6.9 Hz, 1H), 2.44-2.35 (m, 2H), 2.01-1.90 (m, 2H), 1.73-1.57 (m, 3H), 1.42 (s, 9H), 1.29 (q, *J* = 6.6, 5.9 Hz, 1H).

**¹³C-NMR** (101 MHz, MeOH-d₄) δ [ppm] = 177.3, 174.3, 174.0, 157.9, 152.8, 149.7, 145.6, 141.5, 126.5, 123.7, 121.2, 113.7, 80.7, 53.4, 52.7, 52.4, 43.5, 41.7, 39.8, 37.6, 34.8, 32.6, 28.7,24.2.

**HRMS** (ESI): calc. for C₃₀H₄₃O₇N₆⁺ [M+H]⁺: 599.3188, found: 599.3191.

**UV**/**VIS** (LCMS): λₘₐₓ = 412 nm.

***t*_{R}** (LCMS, MeCN/H₂O/formic acid = 90/10/0.1 → 10/90/0.1 over 7 min) = 2.915 min.

In a round bottom flask, dimethyl (2*S*,4*S*)-2-(4-((4-((*E*)-(4-((2-aminoethyl)amino)phenyl)-diazenyl)phenyl)amino)-4-oxobutyl)-4-((*tert*-butoxycarbonyl)amino) pentanedioate (84 mg, 0.13 mmol, 1.0 equiv.) was dissolved in a mixture of H₂O (3.7 mL) and THF (7.3 mL) before lithium hydroxide (78 mg, 3.3 mmol, 25 equiv.) was added as a solid at 0 □C in one portion. The reaction was stirred for 2 h at 0°C, before it was neutralized by addition of formic acid (0.12 mL, 3.3 mmol, 25 equiv.). The THF was removed *in vacuo* before the aqueous phase was subjected to RP column chromatography (100/0 → 75/25 = 1 mM HCl/MeCN). The product containing fractions were combined, concentrated in *vacuo* and freeze-dried to obtain **10** as its red HCl salt (33.5 mg, 55 µmol) in 43% yield.

**¹H**-**NMR** (400 MHz, DMSO-d₆) δ [ppm] 10.18 (s, 1H), 7.80-7.64 (m, 6H), 6.82 (s, 1H), 6.75 (d, *J* = 8.6 Hz, 2H), 6.51 (d, *J* = 7.7 Hz, 1H), 3.91 (q, *J* = 8.2 Hz, 1H), 3.46-3.36 (m, 2H), 3.02 (t, *J* = 6.3 Hz, 2H), 2.47-2.37 (m, 1H), 2.32 (t, *J=* 7.2 Hz, 2H), 1.72-1.44 (m, 5H), 1.37 (s, 9H), 1.34 (s, 1H).

**¹³C**-**NMR** (101 MHz, DMSO-d₆) δ [ppm] = 176.9, 174.7, 171.8, 155.3, 151.5, 148.2, 143.8, 141.3, 125.2, 123.0, 119.7, 112.4, 78.2, 52.9, 42.4, 40.7, 38.3, 37.0, 35.8, 31.6, 28.7, 23.6. **HRMS** (ESI): calc. for C₂₈H₃₉O₇N₆⁺ [M+H]⁺: 571.2875, found: 571.2877.

**UV/VIS** (LCMS): λₘₐₓ = 412 nm.

***t*_{R}** (LCMS, MeCN/H₂O/formic acid = 90/10/0.1 → 10/90/0.1 over 7 min) = 2.438 min.

### (2S,4S)-2-(4-((4-((E)-(4-((1-azido-39-oxo-3,6,9,12,15,18,21,24,27,30,33,36-dodecaoxa-40-azadotetracontan-42-yl)amino)phenyl)diazenyl)phenyl)amino)-4-oxobutyl)-4-((tert-butoxycarbonyl)amino)pentanedioic acid, (18)

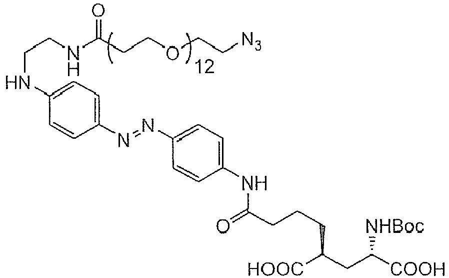

**18** was prepared according to general procedure A.

### Amounts:

N₃-PEG₁₂-NHS-Ester (Baseclick #BCL-033, 10.0 mg, 13.5 µmol, 1.0 equiv.)
**10** (8.5 mg, 14.9 µmol, 1.1 equiv.)
DIPEA (3.5 mg, 27.0 µmol, 4.7 µL, 2.0 equiv.)

yield: 14.0 mg (11.7 µmol, 87%), red-orange solid.

**HRMS (ESI):** calc. for C₅₅H₈₇N₉O₂₀²⁻⁻ [M-2H]²⁻: 596.8039, found: 596.8035.

**UV/Vis** (LCMS): λₘₐₓ = 412 nm.

***t*_{R}** (LCMS; MeCN/H₂O/formic acid = 10/90/0.1 → 90/10/0.1 over 10 min) = 5.117 min.

### (2S,4S)-2-amino-4-(4-((4-((E)-(4-((1-(8-(4-((4-((2-amino-9H-purin-6-yl)oxy)methyl)-benzyl)amino)-4-oxobutanoyl)-8,9-dihydro-1H-dibenzo[b,f][1,2,3]triazolo[4,5-d]azo-ein-1-yl)-39-oxo-3,6,9,12,15,18,21,24,27,30,33,36-dodecaoxa-40-azadotetra-contan-42-yl)amino)phenyl)diazenyl)phenyl)amino)-4-oxobutyl)pentanedioic acid, BGAG_{12,460}

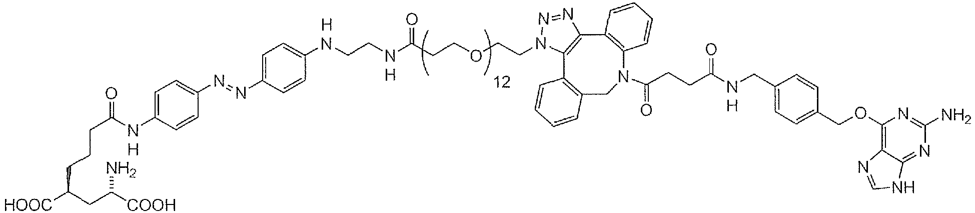

In a round bottom flask, 5 (1.0 mg, 1.79 µmol) and **18** (2.1 mg, 1.79 µmol) were combined and dissolved in MeOH. After stirring at r.t. for 30 min all starting material was consumed according to LCMS and all volatiles were removed *in* v*acuo* to obtain the crude protected triazole. The solid was treated with 0.35 mL neat TFA for 10 min at r.t. before Et₂O was added and the suspension was subjected to sedimentation (4,000 rpm, r.t., 20 min) to collect a deep-red solid, which was washed again with Et₂O and dried under HV to obtain 1.2 mg (1.03 µmol) **BGAG₁₂**(460) in 58% yield (over 2 steps).

**HRMS (ESI):** calc. for C₈₂H₁₁₀N₁₆O₂₁²⁺ (M+2H)²⁺: 827.4010, found: 827.4015.

**UV/Vis** (LCMS): λₘₐₓ = 413 mm.

***t*_{R} (LCMS; MeCN/H₂O/formic acid = 10/90/0.1 → 90/10/0.1 over 10 min) = 3.238 min.**

### (2S,4S)-2-Amino-4-(4-oxo-4-((4-((E)-(4-(38-oxo-2,5,8,11,14,17,20,23,26,29,32,35-dodecaoxa-39-azahentetracontan-41-amido)phenyl)diazenyl)phenyl)amino)butyl)-pentanedioic acid, D-AG₁₂

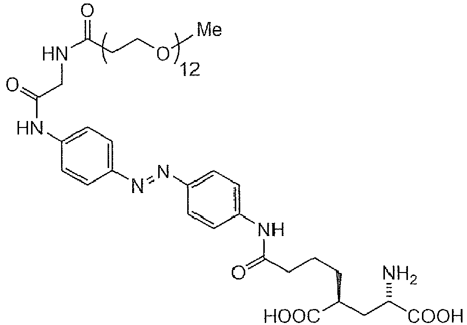

**D-AG12** was prepared according to general procedures A and C.

### Amounts:

Methyl-PEG₁₂-NHS-ester (Thermo Scientific #22685, 9.7 mg, 14 µmol, 1.0 equiv.)
**8** (9.1 mg, 16 µmol, 1.1 equiv.)
DIPEA (4.9 µL, 28 µmol, 2 equiv.) yield: 8.0 mg (7.6 µmol, 54% over two steps), orange solid.

**1H-NMR** (400 MHz, DMSO-d₆) δ [ppm] = 10.31 (s, 1H), 10.27 (s, 1H), 8.27 (t, *J=* 5.8 Hz, 1H), 7.88-7.76 (m, 8H), 3.93 (d, *J* = 5.7 Hz, 2H), 3.63 (t, *J=* 6.5 Hz, 5H), 3.50 (s, 45H), 3.23 (s, 4H), 2.61 (s, 1 H), 2.43 (t, *J* = 6.5 Hz, 5H), 2.36 (t, *J* = 7.0 Hz, 2H), 1.83 (s, 1H), 1.60 (p, *J* = 11.4, 10.7 Hz, 3H), 1.48-1.39 (m, 1H).

**HRMS** (ESI): calc. for C₄₉H₇₉N₆NaO₁₉²⁺ [M+Na+H]²⁺: 539.2643, found: 539.2639. **UV/VIS** (LCMS): λₘₐₓ (π → π□) = 368 nm.

***t*_{R}** (LCMS, MeCN/H₂O/formic acid = 90/1010.1 → 10/90/0.1 over 10 min) = 3.346 min.

### (2S,4S)-2-Amino-4-(4-oxo-4-((4-((E)-(4-((38-oxo-2,5,8,11,14,17,20,23,26,29,32,35-dodecaoxa-39-azahentetracontan-41-yl)amino)phenyl)diazenyl)phenyl)amino)-butyl)pentanedioic acid, D-AG₁₂₍₄₄₅₎

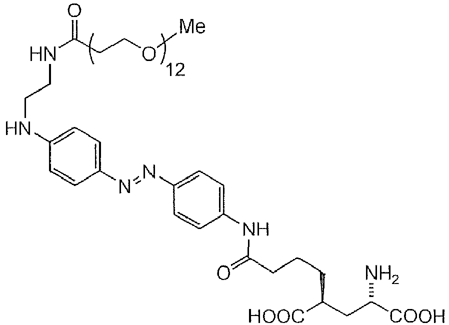

***D*-AG₁₂₍₄₄₅₎** was prepared according to general procedures A and C.

### Amounts:

Methyl-PEG12-NHS-ester (Thermo Scientific, #22685, 9.8 mg, 14 µmol, 1.0 equiv.)
**18** (9.0 mg, 16 µmol, 1.1 equiv.)

yield: 12 mg, (12 µmol, 73% yield over two steps), red solid.

**¹H NMR** (400 MHz, DMSO-d₆) δ [ppm] = 10.17 (s, 1H), 8.03 (t, *J* = 5.5 Hz, 1H), 7.71 (dt, *J* = 12.6, 9.1 Hz, 6H), 6.70 (d, *J=* 8.8 Hz, 2H), 6.62 (s, 1H), 3.62 (dt, *J=* 18.7, 7.1 Hz, 4H), 3.49 (d, *J* = 3.0 Hz, 50H), 2.60 (q, *J* = 7.3 Hz, 1H), 2.34 (q, *J* = 6.6 Hz, 5H), 1.95-1.74 (m, 2H), 1.68-1.37 (m, 5H).

**HRMS** (ESI): calc. for C₄₉H₈₁N₆NaO₁₈²⁺ [M+Na+H]⁺: 532.2747, found: 532.2743. **UV/VIS** (LCMS): λₘₐₓ = 412 nm.

***t*_{R}** (LCMS, MeCN/H₂O/formic acid = 90/10/0.1 → 10/90/0.1 over 10 min) = 3.397 min.

### 1.3 HEK293T and Hippocampal Neuron Electrophysiology

HEK 293T cell recordings were performed as described previously (Levitz et al., 2013). Cells were seeded on 18 mm glass coverslips and transfected with 0.7 µg/well SNAP-mGluR2 (and/or LiGluR: GluK2-L439C) and GIRK1-F137S DNA, along with 0.1 µg/well tdTomato as a transfection marker, using Lipofectamine 2000 (Invitrogen). Whole-cell HEK cell recordings were performed 24-48 hrs later at room temperature (22-24 °C) using an Axopatch 200B headstage/amplifier (Molecular Devices) on an inverted microscope (Olympus IX series) or a EPC10 USB patch clamp amplifier (HEKA) and PatchMaster software (HEKA) on a Leica DM IL LED. Recordings were performed in high potassium (HK) extracellular solution containing (in mM): 120 KCl, 29 NaCl, 1 MgCl₂, 2 CaCl₂, 10 Hepes, pH 7.4. Glass pipettes of resistance between 4 and 8 Ω M were filled with intracellular solution containing (in mM): 140 KCl, 10 Hepes, 3 Na₂ATP, 0.2 Na₂GTP, 5 EGTA, 3 MgCl₂, pH 7.4. Voltage-clamp recordings were typically performed at -60 mV. Drugs were purchased from Tocris, diluted in HK solution and applied using a gravity-driven perfusion system. Data were analyzed with Clampfit (Molecular Devices) or IgorPro (v6.22, wavemetrics).

Prior to recording, cells were washed with extracellular labeling solution and labeled with BGAG variants at the reported concentrations for 45-50 minutes in an incubator at 37 °C. The extracellular labeling solution contained (in mM): 138 NaCl, 1.5 KCl, 1 MgCl₂, 2 CaCl₂, 10 HEPES, pH 7.4. For overnight labeling experiments, BGAG was diluted in HEK cell culture media (DMEM + 5% FBS). For experiments involving LiGluR, following BGAG incubation cells were incubated for 5 minutes at room temperature with 0.3 mg/mL concavalin A to prevent receptor desensitization followed by 50 µM L-MAG0₄₆₀ for 30 minutes at room temperature. Illumination was mediated by Xe-lamp (DG4, Sutter) in combination with excitation filters. Neutral density filters (Omegafilters) were used to vary the light intensity.

Dissociated hippocampal neuron cultures were prepared from postnatal P0 or P1 mice on 12 mm glass coverslips as previously described (Levitz et al., 2013). Neurons were transfected with SNAP-mGluR2 (1.5 µg/well) and tdTomato (0.25 µg/well as a transfection marker) using the calcium phosphate method at DIV9. Whole cell patch clamp experiments were performed 3-6 days after transfection (DIV 12-15). Labeling was performed using the same protocol as HEK cells except BGAG was diluted in extracellular recording solution containing (in mM): 138 NaCl, 1.5 KCl, 1.2 MgCl₂, 2.5 CaCl₂, 10 glucose, 5 HEPES, pH 7.4. Glass pipettes of resistance 4-8 MΩ were filled with an intracellular solution containing (in mM): 140 K-gluconate, 10 NaCl, 5 EGTA, 2 MgCl₂, 1 CaCl₂, 10 HEPES, 2 MgATP and 0.3 Na₂GTP, pH 7.2. Autaptic neurons were voltage clamped at -60 mV and a 2-3 ms voltage step to +20 mV was used to evoke a spike followed (~3-5 ms later) by an EPSC. Stimulation was performed once every 12 s to prevent rundown.

Confocal imaging of SNAP-mGluR2-GFP and Alexa dye-labeled constructs was performed on a Zeiss LSM780 AxioExaminer. Dye labeling was performed in appropriate extracellular solutions for 45 minutes at 1 µM in an incubator at 37 °C and extensively washed before imaging.

### Example 2 Results

### 2.1 Design of PORTL photoswitches for metabotropic glutamate receptors

mGluRs are class C GPCRs that mediate many aspects of glutamatergic signaling in the brain and serve as drug targets for a number of neurological disorders (see e.g. Conn et al., 1997). The defining structural feature of class C GPCRs is a large, bi-lobed extracellular ligand-binding domain (LBD) that assembles as a dimer and mediates receptor activation. We previously developed photoswitchable versions of mGluR2, 3, and 6, termed "LimGluRs", via cysteine-conjugation of D-Maleimide-Azobenzene Glutamate ("D-MAG") molecules to the LBD near the glutamate binding site (Levitz et al., 2013; Carroll et al., 2015). In addition, previous work has shown that *N*-terminal SNAP-tagged mGluRs retain normal function and may be efficiently labeled in living cells (Doumazane et al., 2013). In order to take advantage of the many attractive properties of the SNAP-tag linkage relative to that of cysteine-maleimide, we sought to optically control the LBD of mGluR2 via PORTL conjugation to a genetically-encoded SNAP-tag fused to the LBD.

To design a new family of photoswitches we first analyzed SNAP and mGluR crystal structures to determine the dimensions required to permit a compound conjugated to an *N-*terminal SNAP-tag via a BG group at one end to reach the orthosteric binding site within the mGluR LBD via a glutamate at the other end (Figure 2a). We decided to place the central photoswitchable azobenzene unit close to the glutamate ligand based on the logic that the ability of the glutamate moiety to dock in the binding pocket and activate mGluR2 would be altered by photo-isomerization of the azobenzene, as achieved earlier for soluble photochromic ligands (see e.g. Volgraf et al., 2006; Broichhagen et al., 2014; Damijonaitis et al., 2015) rather than a length-dependent change in the ability to reach the binding site. Furthermore, we hypothesized that a long, flexible polyethylene glycol linker between the BG and azobenzene units would span the necessary distance between the SNAP domain and the mGluR2 LBD and permit the glutamate moiety to reach the binding site (Figure 2b).

Based on our previous work, which indicated that agonism of mGluR2 via glutamate- azobenzene molecules requires 4' D stereochemistry, which we refer to as "D-MAG" (Levitz et al., 2013), we decided to maintain this feature in our new SNAP-tag photoswitches. We opted to construct the linker between BG and azobenzene out of monodisperse PEG-polymers of different sizes. PEG polymers do not strongly adhere to protein surfaces and are known to be conformationally very flexible. To allow this system to be adopted for other pharmacophores in the future, we designed the synthetic chemistry to be flexible as well, using amide bond formation and click chemistry for rapid assembly. Alkyne-azide click chemistry has been extensively used for bioorthogonal reactions and can be employed in presence of benzylguanines (see e.g. Song et al., 2015). Both the Cu(I)-catalyzed click chemistry or the cyclooctyne strain promoted version, which can be used *in vivo*, are available.

Together, these considerations led to the design of two families of benzylguanine-azoglutamates with either a diacyl azidianiline switch (**BGAGₙ**), as used in the original set of D-MAGs for a 2-wavelength on/off bistable optical control of mGluRs^{10g}, or a red-shifted azobenzene switch (**BGAG**_{**n**(460)}), as used more recently for single wavelength single or two-photon control of an mGluR (Carroll et al., 2015; Kienzler et al., 2013) (Figure 2c). In these molecules, the first index denotes the number of ethylene glycol repeat units and the tether length, whereas the number in brackets indicated the wavelength that results in maximum *cis-*azobenzene content upon irradiation.

### 2.2 Synthesis of Benzylguanines-Azobenzenes-Glutamates (BGAGs)

Our synthesis of BGAGs started with guanine derivative **1**, which was converted into the known benzylguanine (**BG**) in 5 steps (Keppler et al., 2003) (Figure 3a). Coupling with pent-3-yenoic acid **(2)** then yielded BG-alkyne **4**. Alternatively, cyclooctyne **4** was linked to **BG** by amidation to obtain BG-DBCO, **5**.

On the ligand side, we utilized several steps from the reported synthesis of D-MAGs starting from L-glutamate (Levitz et al., 2013) to synthesize glutamate azobenzene **6**. Acylation with glycine derivative **7**, followed by deprotection, gave primary amine **8**, whereas reductive amination with aldehyde **9** (Chen et al., 2007) and deprotection yielded diamine **10**. (Figure 3b).

Coupling of both **8** and **10** with bifunctional PEG-O-Su esters of varying length yielded azides **15-18** (whereas **14** was obtained by HBTU-mediated coupling) that were ready for click chemistry (Figure 3c).

**BGAGs** with a "regular" azobenzene switch were synthesized by Cu(I) catalyzed azide alkyne click chemistry, followed by deprotection, which yielded **BGAG**₀, _{4, 8 and 12} (Figure 3d). It should be noted, that high temperatures and high catalyst loadings were needed to drive the click-reaction to completion and that red-shifted version could not be obtained from **18** and **3** under these conditions. Therefore, strain promoted reaction of **18** with 5, followed by deprotection of the amino acid moiety, was used instead, which gave the red-shifted photoswitch **BGAG**_{**12**(460)}.

### 2.3 Optical control of SNAG-mGluR2 in HEK293T cells

After synthesizing the set of BGAG molecules, we next sought to test whether they could be efficiently conjugated to SNAP-mGluR2 and used to optically manipulate mGluR2 function (Figure 2d).

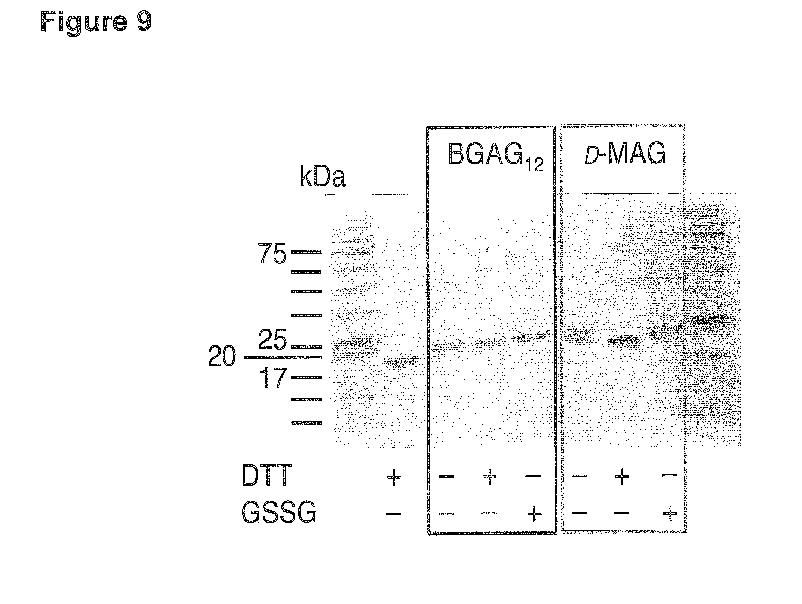

We first expressed a GFP-fusion construct (SNAP-mGluR2-GFP) in HEK293T cells and saw efficient labeling with a BG-conjugated Alexa dye that was limited to the cell surface (Figure 8), as previously reported (Doumzane et al., 2013; Vafabakhsh et al.; 2015). This indicated that BG-conjugated compounds are unlikely to cross the membrane and will, thus, primarily target receptors on the cell surface. Furthermore, *in vitro* studies showed, unlike maleimides, no dependence of SNAP labeling on the presence of a reducing or oxidizing environment (Figure 9).

We next tested the ability of BGAGs to photoactivate SNAP-mGluR2 using whole cell patch-clamp in HEK293T cells co-transfected with the G protein-activated inward rectifier potassium (GIRK) channel. Cells expressing SNAP-mGluR2 were initially incubated with 10 µM **BGAG₁₂** for 45 minutes at 37 °C. Following extensive washing to remove excess, non-attached photoswitches, photoisomerization to the *cis* configuration with a brief (<1 s) bout of illumination at 380 nm produced robust photoactivation that persisted in the dark and was reversed by a brief (~1 s) bout of illumination at 500 nm to isomerize the azobenzene back to the *trans* state (Figure 4a, Figure 10a). mGluR2 photoactivation *via* **BGAG₁₂** was highly reproducible. In the photoswitch "off" state (i.e. in the dark or following illumination at 500 nm), responses to the native neurotransmitter ligand glutamate was intact. Photocurrents were abolished at high glutamate concentrations, suggesting that **BGAG₁₂** does not function as a partial agonist (Figure 4a). Light responses were ~60% of the responses to saturating glutamate (59.3 ± 2.8%, n=10 cells), consistent with both efficient conjugation and receptor activation. Importantly, cells expressing wild type mGluR2 (i.e. with no SNAP tag) and incubated with **BGAG₁₂** showed no light responses (Figure 10b). Given the successful optical control of mGluR2, we termed the tool that combines SNAP-mGluR2 and BGAG "SNAG-mGluR2". SNAG-mGluR2 showed similar photocurrent efficacy and kinetics to the previously reported LimGluR2 (Levitz et al., 2013). SNAG-mGluR2 photoactivation was fully blocked by the competitive mGluR2 antagonist LY341495, without altering the baseline current, supporting the interpretation that **BGAG₁₂** activates mGluR2 *via* its native, orthosteric binding site and does not significantly activate in the *trans* configuration of the azobenzene (Figure 10c). The apparent affinity for glutamate of SNAG-mGluR2 was comparable to that of both SNAP-mGluR2 not labeled by **BGAG₁₂** and, indeed, of wild type mGluR2 (Figure 10d), indicating that normal mGluR2 function is maintained.

We next tested different labeling conditions of **BGAG₁₂** and found that 45 minute incubation with ≥ 1 µM **BGAG₁₂** showed optimal labeling (Figure 11a and b). However, photocurrents were still observed with 100 nM labeling for 45 minutes (Figure 11c) and these were increased with overnight labeling at 100 nM and could even be observed with concentrations as low as 10 nm with overnight labeling (Figures 11d-f). Remarkably, the labeling solution could be reused for multiple experiments for one week following dilution in aqueous buffer at room temperature, without a decline in efficacy of optical activation (Figure 4b, c). This result is in stark contrast to maleimide-based MAG photoswitches, which typically need to be applied at concentrations up to 100-200 µM (Levitz et al., 2013; Gorostiza et al., 2007) and are hydrolyzed in water with a half-life in the range of minutes to hours (Hermanson 2013).

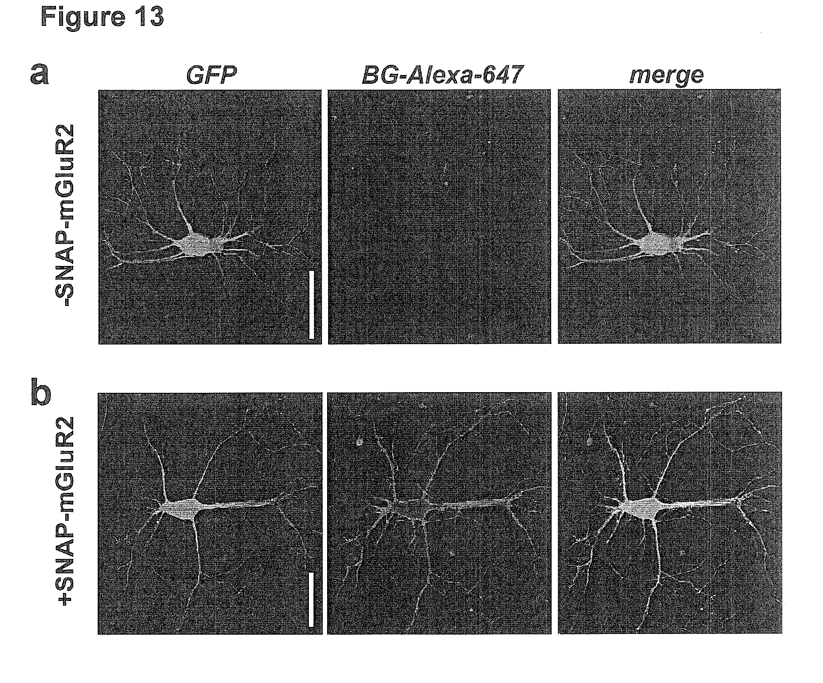

To further explore the mechanism of photoswitching in SNAG-mGluR2, we synthesized a PCL version of **BGAG₁₂** where the BG group was omitted ("AG₁₂"; Figure 12a). AG₁₂ photoagonized SNAP-mGluR2 with the same directionality as **BGAG₁₂** (Figure 12b), supporting the hypothesis that photoswitching is based on the relative efficacy of the azobenzene-glutamate moiety in *cis* versus *trans*, rather than a length or geometry-dependent change in the ability to reach the binding site. We also tested BGAG variants ranging in length from 0 to 8 PEG repeats and found comparable photoactivation of SNAG-mGluR2 to **BGAG₁₂** for all versions (Figure 13), suggesting similar effective concentrations of the ligand near the binding pocket.

We next tested the red-shifted version of **BGAG₁₂**, **BGAG**_{**12**(460)}, to see if we could develop a SNAG-mGluR2 variant that is controlled with a single wavelength of visible light. Following labeling with 10 µM **BGAG**_{**12**(460)} photoactivation of SNAP-mGluR2 was achieved reproducibly in response to illumination with blue light (420-470 nm bandpass) (Figure 4d). Relaxation occurred rapidly in the dark following illumination, as expected, and the photoactivation was ~35% relative to saturating glutamate (34.9 ± 4.2%, n=18 cells). We termed the combination of SNAP-mGluR2 and **BGAG**_{**12**(460)} "SNAG₄₆₀-mGluR2".

Having developed multiple versions of SNAG-mGluR2 that were able to efficiently photoactivate mGluR2, we next tested if this toolset could be used to optically manipulate mGluR2 in its native neuronal setting.

### 2.4 Optical manipulation of excitability and synaptic transmission via SNAG-mGluR2 in hippocampal neurons

mGluR2, like other neuronal Gi/o-coupled GPCRs, primarily signals either somatodendritically, to hyperpolarize membranes through the activation of GIRK channels, or presynaptically, to inhibit neurotransmitter release by a number of mechanisms, including inhibition of voltage-gated calcium channels (Conn et al., 1997). We hypothesized that SNAG-mGluR2 would efficiently gate both of those canonical functions in neurons.

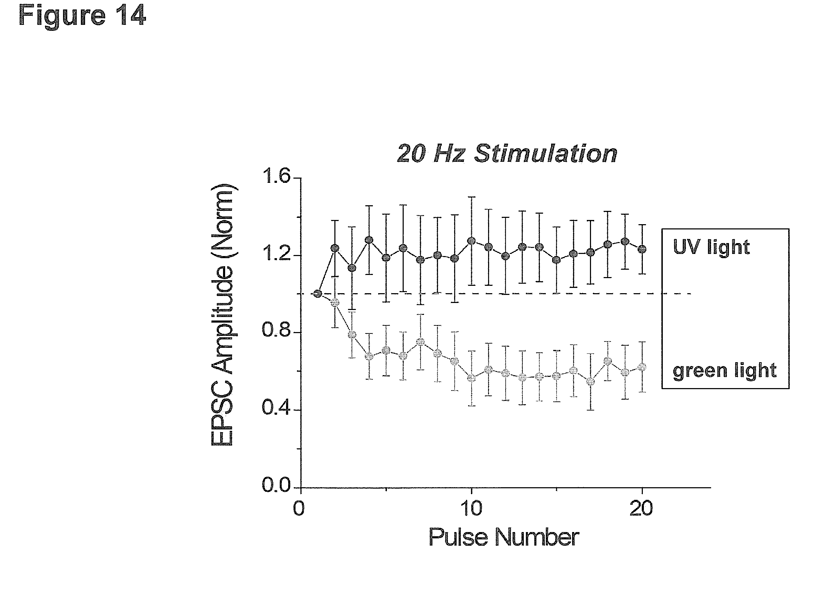

We first expressed SNAP-mGluR2-GFP in dissociated hippocampal neurons and labeled with BG-Alexa-647 to determine if SNAP-BG conjugation could occur efficiently in neuronal cultures, which are considerably denser than HEK 293T cell cultures. We observed strong SNAP-mGluR2-GFP expression and surface labeling with Alexa-647 (Figure 5a), indicating that the SNAP tethering approach is suitable to neurons. Importantly, untransfected cells did not show BG-Alexa-647 fluorescence (Figure 14), confirming the specificity of the labeling chemistry. Next, instead of labeling with BG-Alexa-647, we labeled with **BGAG₁₂** and observed rapid membrane hyperpolarization (~2-8 mV) in response to illumination at 380 nm, which was reversed by illumination at 500 nm (Figure 5b). When the neurons were at depolarized potentials that induced firing, the light-induced hyperpolarization was sufficient to inhibit the action potentials (Figure 5c).

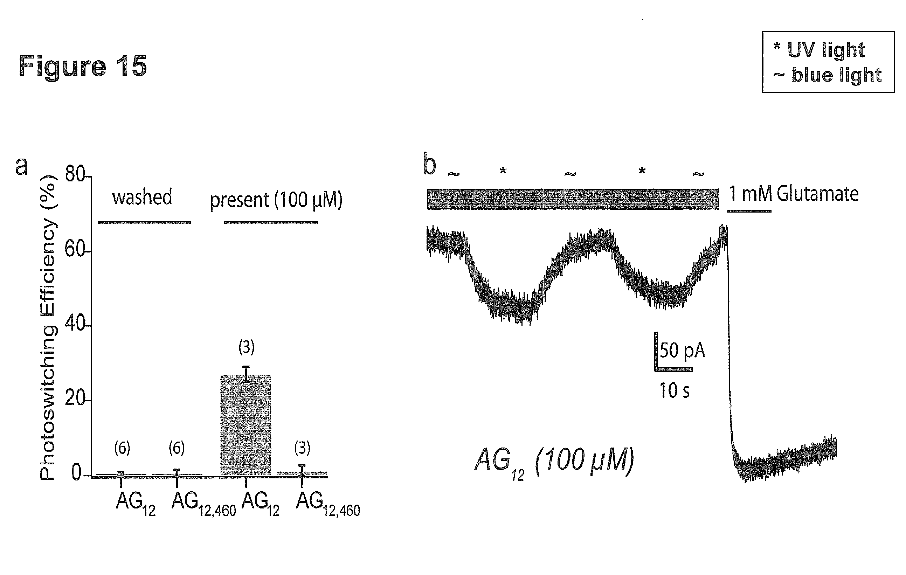

To test for presynaptic inhibition, we cultured hippocampal neurons at low density to promote the formation of autapses, *i.e.* synapses between the axon and dendrites of the same neuron. In autaptic neurons, photoactivation of SNAG-mGluR2 reversibly decreased excitatory post synaptic current (EPSC) amplitude by up to 70% (average = 48.3 ± 7.3%, n=5 cells) (Figures 5d, e). Optical inhibition of EPSC amplitude was accompanied by an increase in paired pulse ratio (Figures 5f, g) and a decrease in synaptic depression during high frequency trains (Figure 15), consistent with a presynaptic reduction in the probability of transmitter release. Together, these observations demonstrate that the SNAG system is well suited for neuronal cells and that SNAG-mGluR2 itself is a powerful tool for optical manipulation of neuronal inhibition *via* native mGluR2-mediated mechanisms that control neural firing and transmitter release.

### 2.5 Dual optical control of SNAG-mGluR2 and LiGluR via orthogonal photoswitch labeling

A major goal in physiology is to be able to manipulate independently different receptors within the same preparation using different wavelengths of light. This type of experiment could be extremely powerful for deciphering the different roles, and potential cross-talk, of different signaling pathways within a cell or neural circuit. With this goal in mind, we wondered if SNAG-mGluR2 could be used in conjunction with a previous generation photoswitchable receptor to provide individual optical control of two receptors within the same cell. We turned to LiGluR, a GluK2 ionotropic glutamate receptor that is photoactivated by molecules of the maleimide-azobenzene-glutamate (MAG) family through cysteine-maleimide linkage (Volgraf et al., 2006; Reiner et al., 2015).

To test this, we co-expressed SNAP-mGluR2 along with its GIRK channel effector and LiGluR (GluK2-L439C) in HEK293T cells. We labeled the cells with **BGAG₁₂** for 30 minutes, and then with L-MAG0₄₆₀, a blue light-activated, spontaneously relaxing version of MAG with similar spectral properties to **BGAG**_{**12**(460)} (Kienzler et al., 2013). Due to the spectral and light sensitivity differences between the two photoswitches, we were able to independently and sequentially activate SNAG-mGluR2 and LiGluR (Figure 6a). Photoactivation of SNAG-mGluR2 with dim illumination at 380 nm induced slow inward photocurrents, which were deactivated by illumination at 590 nm, as shown above. 590 nm yellow light was used to ensure orthogonality to L-MAG0₄₆₀. In contrast, photoactivation of LiGluR-L-MAG0₄₆₀ by illumination at 500 nm induced rapid, spontaneously-relaxing photocurrents, as shown earlier (Kienzler et al., 2013). When only one of the receptors was expressed, only its characteristic photo-response was seen. In the case of SNAG-mGluR2 this was a slow ON, slow OFF photocurrent induced by illumination at 380 nm and 500 nm, respectively, whereas in the case of LiGluR-L-MAG0₄₆₀ this was a rapid, spontaneously-relaxing photocurrent, which was triggered by illumination at 500 mm, which turned off spontaneously in the dark (Figures 6b, c).

Together these experiments show that the PORTL approach based on conjugation of BGAGs to SNAP-tagged receptors allows for independent, dual optical control within the same preparation, a major step forward for chemical optogenetics.

The features disclosed in the foregoing description, in the claims and/or in the accompanying drawings may, both separately and in any combination thereof, be material for realizing the invention in diverse forms thereof.

### REFERENCES

Banghart, M.; Borges, K.; Isacoff, E.; Trauner, D.; Kramer, R. H., Light-activated ion channels for remote control of neuronal firing. Nat Neurosci 2004, 7 (12), 1381-6.
Bojkowska, K.; Santoni de Sio, F.; Barde, I.; Offner, S.; Verp, S.; Heinis, C.; Johnsson, K.; Trono, D., Measuring in vivo protein half-life. Chemistry & biology 2011, 18 (6), 805-15
Broichhagen, J.; Schonberger, M.; Cork, S. C.; Frank, J. A.; Marchetti, P.; Bugliani, M.; Shapiro, A. M.; Trapp, S.; Rutter, G. A.; Hodson, D. J.; Trauner, D., Optical control of insulin release using a photoswitchable sulfonylurea. Nat Commun 2014, 5, 5116.
Broichhagen, J.; Trauner, D., The in vivo chemistry of photochromic tethered ligands. Current Opinion in Chemical Biology 2014, 21, 121-127.
Carroll, E. C.; Berlin, S.; Levitz, J.; Kienzler, M. A.; Yuan, Z.; Madsen, D.; Larsen, D. S.; Isacoff, E. Y., Two-photon brightness of azobenzene photoswitches designed for glutamate receptor optogenetics. Proc Natl Acad Sci U S A 2015, 112 (7), E776-85.
Chen, J. J.; Aduda, V., DMSO-aided o-iodoxybenzoic acid (IBX) oxidation of Fmoc-protected amino alcohols. Synthetic Commun 2007, 37 (19-21), 3493-3499.
Conn, P. J.; Pin, J. P., Pharmacology and functions of metabotropic glutamate receptors. Annu Rev Pharmacol Toxicol 1997, 37, 205-37.
Damijonaitis, A.; Broichhagen, J.; Urushima, T.; Hull, K.; Nagpal, J.; Laprell, L.; Schonberger, M.; Woodmansee, D. H.; Rafiq, A.; Sumser, M. P.; Kummer, W.; Gottschalk, A.; Trauner, D., AzoCholine enables optical control of alpha 7 nicotinic acetylcholine receptors in neural networks. ACS Chem Neurosci 2015.
Di Antonio, M. et al. Selective RNA versus DNA G-quadruplex targeting by situ click chemistry. Angew. Chemie - Int. Ed. 51, 11073-11078 (2012).
Doumazane, E.; Scholler, P.; Fabre, L.; Zwier, J. M.; Trinquet, E.; Pin, J. P.; Rondard, P., Illuminating the activation mechanisms and allosteric properties of metabotropic glutamate receptors. Proc Natl Acad Sci USA 2013, 110 (15), E1416-25.
Fehrentz, T.; Schonberger, M.; Trauner, D., Optochemical genetics. Angew Chem Int Ed Engl 2011, 50 (51), 12156-82.
Fortin, D. L.; Banghart, M. R.; Dunn, T. W.; Borges, K.; Wagenaar, D. A.; Gaudry, Q.; Karakossian, M. H.; Otis, T. S.; Kristan, W. B.; Trauner, D.; Kramer, R. H., Photochemical control of endogenous ion channels and cellular excitability. Nat Methods 2008, 5 (4), 331-8.
Gaub, B. M.; Berry, M. H.; Holt, A. E.; Reiner, A.; Kienzler, M. A.; Dolgova, N.; Nikonov, S.; Aguirre, G. D.; Beltran, W. A.; Flannery, J. G.; Isacoff, E. Y., Restoration of visual function by expression of a light-gated mammalian ion channel in retinal ganglion cells or ON-bipolar cells. Proc Natl Acad Sci U S A 2014, 111 (51), E5574-83.
Gautier, A.; Juillerat, A.; Heinis, C.; Correa, I. R., Jr.; Kindermann, M.; Beaufils, F.; Johnsson, K., An engineered protein tag for multiprotein labeling in living cells. Chemistry & biology 2008, 15 (2), 128-36.
Gorostiza, P.; Volgraf, M.; Numano, R.; Szobota, S.; Trauner, D.; Isacoff, E. Y., Mechanisms of photoswitch conjugation and light activation of an ionotropic glutamate receptor. Proc Natl Acad Sci U S A 2007, 104 (26), 10865-70.
Hermanson, G. T., Bioconjugate Techniques. Third ed.; Academic Press: 2013.
Juillerat, A.; Gronemeyer, T.; Keppler, A.; Gendreizig, S.; Pick, H.; Vogel, H.; Johnsson, K., Directed evolution of O6-alkylguanine-DNA alkyltransferase for efficient labeling of fusion proteins with small molecules in vivo. Chemistry biology 2003, 10 (4), 313-7.
Keppler, A.; Gendreizig, S.; Gronemeyer, T.; Pick, H.; Vogel, H.; Johnsson, K., A general method for the covalent labeling of fusion proteins with small molecules in vivo. Nat Biolechnol 2003, 21 (1), 86-9.
Keppler, A.; Kindermann, M.; Gendreizig, S.; Pick, H.; Vogel, H.; Johnsson, K., Labeling of fusion proteins of O6-alkylguanine-DNA alkyltransferase with small molecules in vivo and in vitro. Methods 2004, 32 (4), 437-44.
Kienzler, M. A.; Reiner, A.; Trautman, E.; Yoo, S.; Trauner, D.; Isacoff, E. Y., A red-shifted, fast-relaxing azobenzene photoswitch for visible light control of an ionotropic glutamate receptor. J Am Chem Soc 2013, 135 (47), 17683-6.
Lemoine, D.; Habermacher, C.; Martz, A.; Mery, P. F.; Bouquier, N.; Diverchy, F.; Taly, A.; Rassendren, F.; Specht, A.; Grutter, T., Optical control of an ion channel gate. Proc Natl Acad Sci USA 2013, 110 (51), 20813-8.
Levitz, J.; Pantoja, C.; Gaub, B.; Janovjak, H.; Reiner, A.; Hoagland, A.; Schoppik, D.; Kane, B.; Stawski, P.; Schier, A. F.; Trauner, D.; Isacoff, E. Y., Optical control of metabotropic glutamate receptors. Nat Neurosci 2013, 16 (4), 507-16.
Lin, W. C.; Davenport, C. M.; Mourot, A.; Vytla, D.; Smith, C. M.; Medeiros, K. A.; Chambers, J. J.; Kramer, R. H., Engineering a light-regulated GABAA receptor for optical control of neural inhibition. ACS chemical biology 2014, 9 (7), 1414-9.
Los, G. V.; Encell, L. P.; McDougall, M. G.; Hartzell, D. D.; Karassina, N.; Zimprich, C.; Wood, M. G.; Learish, R.; Ohana, R. F.; Urh, M.; Simpson, D.; Mendez, J.; Zimmerman, K.; Otto, P.; Vidugiris, G.; Zhu, J.; Darzins, A.; Klaubert, D. H.; Bulleit, R. F.; Wood, K. V., HaloTag: a novel protein labeling technology for cell imaging and protein analysis. ACS chemical biology 2008, 3 (6), 373-82.
Maurel, D.; Comps-Agrar, L.; Brock, C.; Rives, M. L.; Bourrier, E.; Ayoub, M. A.; Bazin, H.; Tinel, N.; Durroux, T.; Prezeau, L.; Trinquet, E.; Pin, J. P., Cell-surface protein-protein interaction analysis with time-resolved FRET and snap-tag technologies: application to GPCR oligomerization. Nat Methods 2008, 5 (6), 561-7.
Reiner, A.; Levitz, J.; Isacoff, E. Y., Controlling ionotropic and metabotropic glutamate receptors with light: principles and potential. Curr Opin Pharmacol 2015, 20, 135-43.
Reymond, L.; Lukinavicius, G.; Umezawa, K.; Maurel, D.; Brun, M. A.; Masharina, A.; Bojkowska, K.; Mollwitz, B.; Schena, A.; Griss, R.; Johnsson, K., Visualizing biochemical activities in living cells through chemistry. Chimia. (Aarau) 2011, 65 (11), 868-71.
Schafer, L. V.; Muller, E. M.; Gaub, H. E.; Grubmuller, H., Elastic properties of photoswitchable azobenzene polymers from molecular dynamics simulations. Angew Chem Int Ed Engl 2007, 46 (13), 2232-7.
Song, X.; Wang, C.; Han, Z.; Xu, Y.; Xiao, Y., Terminal alkyne substituted 06-benzylguanine for versatile and effective syntheses of fluorescent labels to genetically encoded SNAP-tags. RSC Advances 2015, 5, 23646-23649.
Vafabakhsh, R.; Levitz, J.; Isacoff E. Y., Conformational Dynamics of a Class C GPCR. Nature 2015, 524(7566):497-501. doi: 10.1038/nature14679.
Volgraf, M.; Gorostiza, P.; Numano, R.; Kramer, R. H.; Isacoff, E. Y.; Trauner, D., Allosteric control of an ionotropic glutamate receptor with an optical switch. Nat Chem Biol 2006, 2 (1), 47-52.
Wyart, C.; Del Bene, F.; Warp, E.; Scott, E. K.; Trauner, D.; Baier, H.; Isacoff, E. Y., Optogenetic dissection of a behavioural module in the vertebrate spinal cord. Nature 2009, 461 (7262), 407-10.
Yang, G.; de Castro Reis, F.; Sundukova, M.; Pimpinella, S.; Asaro, A.; Castaldi, L.; Batti, L.; Bilbao, D.; Reymond, L.; Johnsson, K.; Heppenstall, P. A., Genetic targeting of chemical indicators in vivo. Nat Methods 2015, 12 (2), 137-9.
Yue, L.; Pawlowski, M.; Dellal, S. S.; Xie, A.; Feng, F.; Otis, T. S.; Bruzik, K. S.; Qian, H.; Pepperberg, D. R., Robust photoregulation of GABA(A) receptors by allosteric modulation with a propofol analogue. Nat Commun 2012, 3, 1095.

## Claims

1. A photo-switchable azobenzene compound having the general formula I
**TAG - link -Azo - L** **(I)**
wherein
**TAG** is a moiety selected from a benzyl guanine (BG), benzyl cytosine (BC), maleimide (M) or alkyl halide (RX),
**link** is a linker group,
**Azo** is the azobenzene moiety, and
**L** is a binding moiety to a target protein.

2. The compound of claim 1, wherein the linker group **link** is a polyethylene glycol (PEG), an alkyl chain or polyamino acid(s),
and/or wherein the binding moiety to the target protein **L** is selected from
- a substrate, an inhibitor, a cofactor, an agonist, an antagonist, a modulator of the target protein,
- a ligand of a receptor of interest (such as an agonist, antagonist, inhibitor, blocker or modulator),
preferably a ligand of a G-protein coupled receptor (GPCR), an ionotropic receptor and/or a receptor-linked enzyme
such as a glutamate, a tetraalkyl ammonium moiety or other pharmaceutical group,
and/or wherein the azobenzene moiety **Azo** is an azobenzene or a derivative thereof, such as heterocyclic azobenzene structure(s), substituted azobenzene structure(s) (e.g. tetra-ortho), bridged azobenzenes (e.g. by an o,o'-ethylene chain).

3. The compound of claim 1 or 2, wherein the activation/deactivation wavelength is in the ultraviolet, visible and infrared light ranges of the electromagnetic spectrum.

4. The compound of any one of claims 1 to 3 having the general formula Ia
**BG - (link)ₙ -Azo - glu** (Ia)
wherein
**BG** is the benzyl guanine moiety,
**link** is a linker group, preferably polyethylene glycol with **n** being 0 to 12, **Azo** is the azobenzene moiety, and
**glu** is a glutamate group.

5. The compound of any one of claims 1 to 4 having one of the formulas of **BGAGₙ** 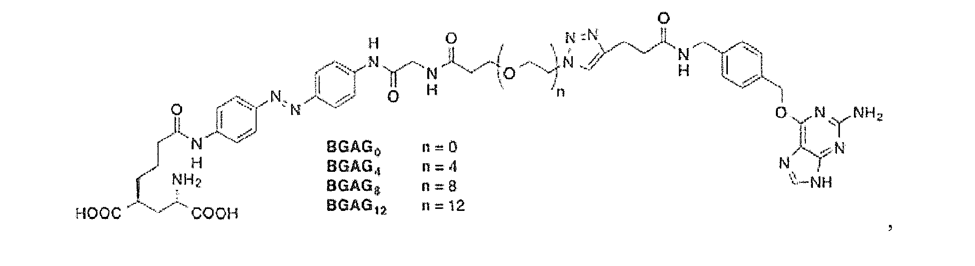 or **BGAG**_{**12**(460)} 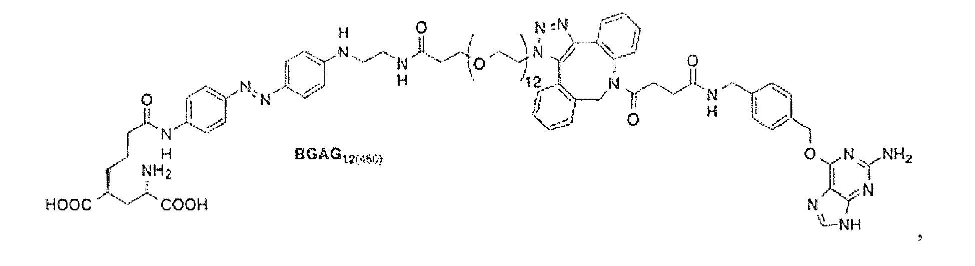 or **BGAG₁₂** 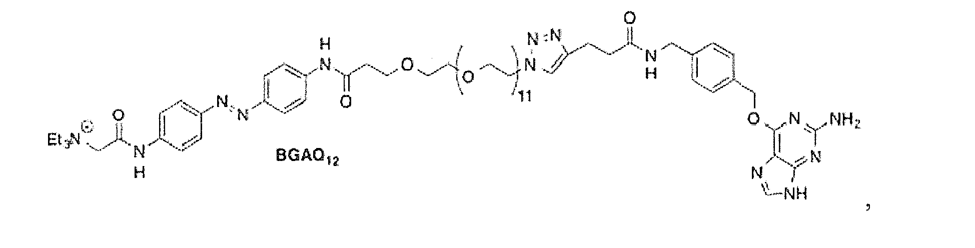 or *L*-**BGAG₁₂** 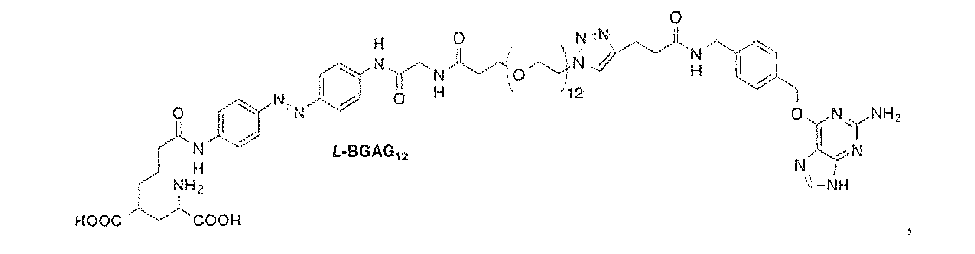 or **BCAG₁₂** 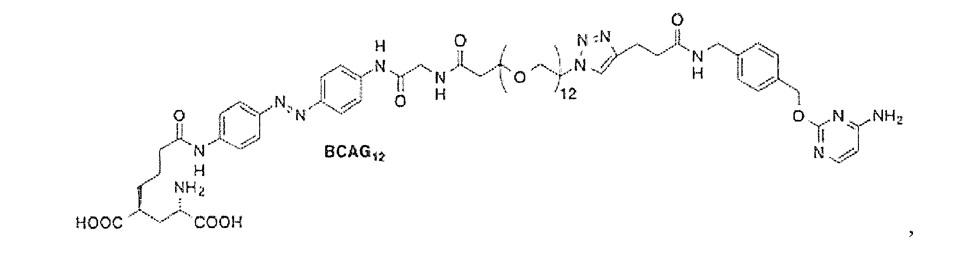 or **CIAG₁₂** 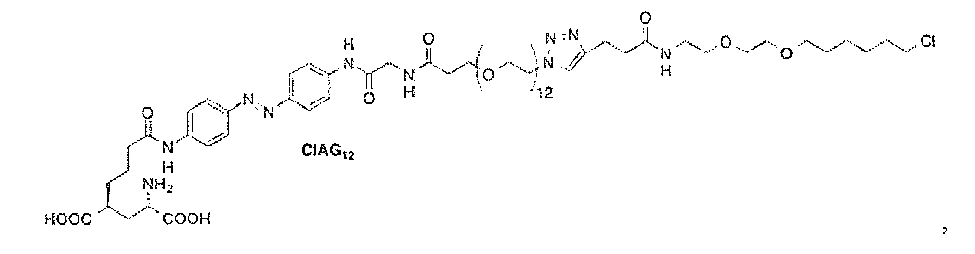 or **BGAG** 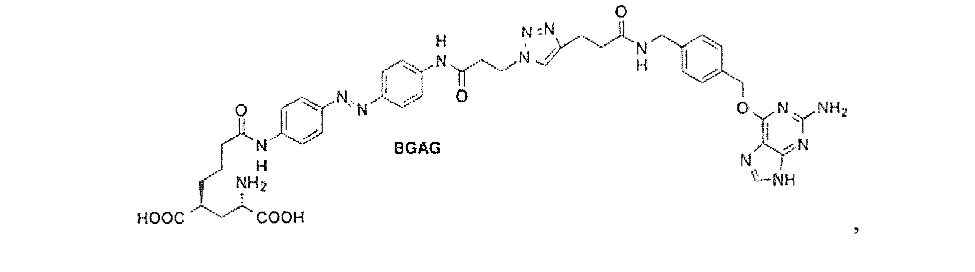 or **BGAP₁₂** 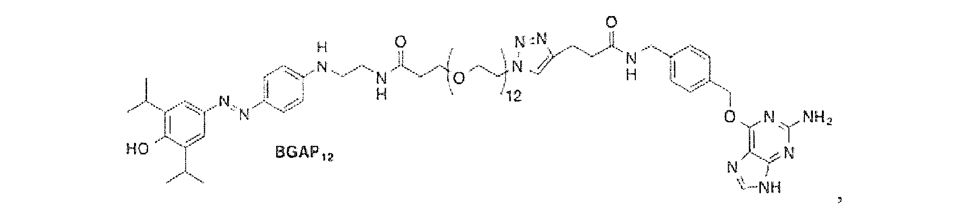 or **BGAF₁₂** 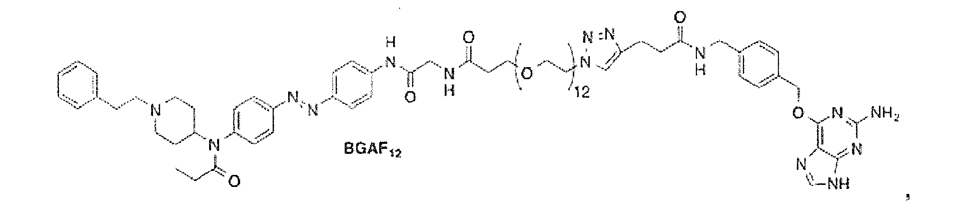 or **BGACh₁₂** 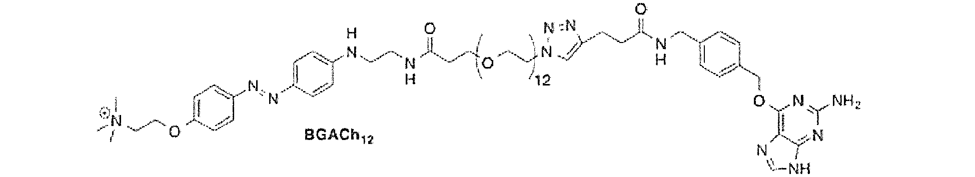 preferably **BGAG₁₂** or **BGAG**_{**12**(460)}.

6. The compound of any one of claims 1 to 5, comprising further group(s) or moiety(ies), such as
- label (e.g. radioisotopes, MRI responsive groups, IR dyes, fluorophores)
- tag(s) (e.g. antibodies, nanobodies, peptides),
- anchoring group(s) (e.g. GPI),
and/or
- other pharmacological units (e.g. fentanyl, GABA, choline, propofol).

7. A pharmaceutical composition, comprising
(i) at least one photo-switchable benzylguanine compound according to any one of claims 1 to 6,
(ii) optionally, pharmaceutically acceptable excipient(s) and/or carrier(s),
(iii) optionally, further compound(s).

8. A photo-sensor protein, comprising
a tag for attaching the photo-switchable azobenzene compound according to any one of claims 1 to 6, and
the photo-switchable azobenzene compound according to any one of claims 1 to 6, which has been attached to the protein via said tag.

9. The photo-sensor protein of claim 8, which is a transmembrane protein or receptor, such as a G protein coupled receptor (GPCR), e.g. a metabotropic glutamate receptor (mGluR2), an ionotropic receptor, ion channel and/or a receptor-linked enzyme,
wherein the tag is preferably selected from a SNAP-tag, a CLIP-tag, or a Halo tag,
wherein preferably said photo-sensor protein is a SNAP-tagged metabotropic glutamate receptor (SNAP-mGluR2), preferably labeled with **BGAG₁₂** or **BGAG**_{**12**(460)}.

10. Use of the photo-switchable azobenzene compound according to any one of claims 1 to 6 for labeling of target proteins,
wherein the target protein is preferably a transmembrane protein or receptor,
such as a G protein coupled receptor (GPCR), e.g. a metabotropic glutamate receptor (mGluR2),
wherein the target protein comprises a tag for labeling which is preferably selected from a SNAP-tag, a CLIP-tag, or a Halo tag.

11. The use of claim 10 for labeling of target proteins, *in vitro*, in intact tissue or *in vivo.*

12. The use of a photo-switchable azobenzene compound according to any one of claims 1 to 6 or a photo-sensor protein of claim 8 or 9 as research tool,
such as:
- for the diagnosis or restoration of vision, sight disorder(s),
- the elucidation of signaling pathways,
- the mapping of neural circuitry,
- the activation/surpression of immune responses,
- the control of nociception
- the control of insulin release and other hormonal pathways, and/or
- as research tool(s) in general.

13. The use of claim 12, comprising the use of a further photo-sensor protein (e.g. using a SNAP-tag),
such as an orthogonal photo-sensor protein (e.g. using a CLIP-tag) or a maleimide-based photo-sensor protein.

14. The photo-switchable azobenzene compound of any one of claims 1 to 6 or the pharmaceutical composition of claim 7 or the photo-sensor protein of claim 8 or 9 for use in medicine.

15. The photo-switchable azobenzene compound of any one of claims 1 to 6 or the pharmaceutical composition of claim 7 or the photo-sensor protein of claim 8 or 9 for use
- in the diagnosis and/or treatment of a sight disorder,
- in the improvement of vision,
- in the improvement of neural disorders,
- in the up-/downregulation of the immune system,
- in the treatment of pain sensation, i.e. nociception, and/or
- in the treatment of hormonal disorders, e.g. diabetes,
optionally comprising the orthogonal optical control of two receptors within the same cell.


SEQUENCE LISTING

<110>  Ludwig-Maximilians-Universität München

<120>  Covalently attached azobenzene switches and their uses for the
       optical control of target proteins

<130>  U30665EP

<160>  2

<170>  PatentIn version 3.5

<210>  1
<211>  872
<212>  PRT
<213>  Rattus norvegicus

<400>  1

Met Glu Ser Leu Leu Gly Phe Leu Ala Leu Leu Leu Leu Trp Gly Ala
1               5                   10                  15

Val Ala Glu Gly Pro Ala Lys Lys Val Leu Thr Leu Glu Gly Asp Leu
            20                  25                  30

Val Leu Gly Gly Leu Phe Pro Val His Gln Lys Gly Gly Pro Ala Glu
        35                  40                  45

Glu Cys Gly Pro Val Asn Glu His Arg Gly Ile Gln Arg Leu Glu Ala
    50                  55                  60

Met Leu Phe Ala Leu Asp Arg Ile Asn Arg Asp Pro His Leu Leu Pro
65                  70                  75                  80

Gly Val Arg Leu Gly Ala His Ile Leu Asp Ser Cys Ser Lys Asp Thr
                85                  90                  95

His Ala Leu Glu Gln Ala Leu Asp Phe Val Arg Ala Ser Leu Ser Arg
            100                 105                 110

Gly Ala Asp Gly Ser Arg His Ile Cys Pro Asp Gly Ser Tyr Ala Thr
        115                 120                 125

His Ser Asp Ala Pro Thr Ala Val Thr Gly Val Ile Gly Gly Ser Tyr
    130                 135                 140

Ser Asp Val Ser Ile Gln Val Ala Asn Leu Leu Arg Leu Phe Gln Ile
145                 150                 155                 160

Pro Gln Ile Ser Tyr Ala Ser Thr Ser Ala Lys Leu Ser Asp Lys Ser
                165                 170                 175

Arg Tyr Asp Tyr Phe Ala Arg Thr Val Pro Pro Asp Phe Phe Gln Ala
            180                 185                 190

Lys Ala Met Ala Glu Ile Leu Arg Phe Phe Asn Trp Thr Tyr Val Ser
        195                 200                 205

Thr Val Ala Ser Glu Gly Asp Tyr Gly Glu Thr Gly Ile Glu Ala Phe
    210                 215                 220

Glu Leu Glu Ala Arg Ala Arg Asn Ile Cys Val Ala Thr Ser Glu Lys
225                 230                 235                 240

Val Gly Arg Ala Met Ser Arg Ala Ala Phe Glu Gly Val Val Arg Ala
                245                 250                 255

Leu Leu Gln Lys Pro Ser Ala Arg Val Ala Val Leu Phe Thr Arg Ser
            260                 265                 270

Glu Asp Ala Arg Glu Leu Leu Ala Ala Thr Gln Arg Leu Asn Ala Ser
        275                 280                 285

Phe Thr Trp Val Ala Ser Asp Gly Trp Gly Ala Leu Glu Ser Val Val
    290                 295                 300

Ala Gly Ser Glu Arg Ala Ala Glu Gly Ala Ile Thr Ile Glu Leu Ala
305                 310                 315                 320

Ser Tyr Pro Ile Ser Asp Phe Ala Ser Tyr Phe Gln Ser Leu Asp Pro
                325                 330                 335

Trp Asn Asn Ser Arg Asn Pro Trp Phe Arg Glu Phe Trp Glu Glu Arg
            340                 345                 350

Phe His Cys Ser Phe Arg Gln Arg Asp Cys Ala Ala His Ser Leu Arg
        355                 360                 365

Ala Val Pro Phe Glu Gln Glu Ser Lys Ile Met Phe Val Val Asn Ala
    370                 375                 380

Val Tyr Ala Met Ala His Ala Leu His Asn Met His Arg Ala Leu Cys
385                 390                 395                 400

Pro Asn Thr Thr His Leu Cys Asp Ala Met Arg Pro Val Asn Gly Arg
                405                 410                 415

Arg Leu Tyr Lys Asp Phe Val Leu Asn Val Lys Phe Asp Ala Pro Phe
            420                 425                 430

Arg Pro Ala Asp Thr Asp Asp Glu Val Arg Phe Asp Arg Phe Gly Asp
        435                 440                 445

Gly Ile Gly Arg Tyr Asn Ile Phe Thr Tyr Leu Arg Ala Gly Ser Gly
    450                 455                 460

Arg Tyr Arg Tyr Gln Lys Val Gly Tyr Trp Ala Glu Gly Leu Thr Leu
465                 470                 475                 480

Asp Thr Ser Phe Ile Pro Trp Ala Ser Pro Ser Ala Gly Pro Leu Pro
                485                 490                 495

Ala Ser Arg Cys Ser Glu Pro Cys Leu Gln Asn Glu Val Lys Ser Val
            500                 505                 510

Gln Pro Gly Glu Val Cys Cys Trp Leu Cys Ile Pro Cys Gln Pro Tyr
        515                 520                 525

Glu Tyr Arg Leu Asp Glu Phe Thr Cys Ala Asp Cys Gly Leu Gly Tyr
    530                 535                 540

Trp Pro Asn Ala Ser Leu Thr Gly Cys Phe Glu Leu Pro Gln Glu Tyr
545                 550                 555                 560

Ile Arg Trp Gly Asp Ala Trp Ala Val Gly Pro Val Thr Ile Ala Cys
                565                 570                 575

Leu Gly Ala Leu Ala Thr Leu Phe Val Leu Gly Val Phe Val Arg His
            580                 585                 590

Asn Ala Thr Pro Val Val Lys Ala Ser Gly Arg Glu Leu Cys Tyr Ile
        595                 600                 605

Leu Leu Gly Gly Val Phe Leu Cys Tyr Cys Met Thr Phe Val Phe Ile
    610                 615                 620

Ala Lys Pro Ser Thr Ala Val Cys Thr Leu Arg Arg Leu Gly Leu Gly
625                 630                 635                 640

Thr Ala Phe Ser Val Cys Tyr Ser Ala Leu Leu Thr Lys Thr Asn Arg
                645                 650                 655

Ile Ala Arg Ile Phe Gly Gly Ala Arg Glu Gly Ala Gln Arg Pro Arg
            660                 665                 670

Phe Ile Ser Pro Ala Ser Gln Val Ala Ile Cys Leu Ala Leu Ile Ser

675                 680                 685

Gly Gln Leu Leu Ile Val Ala Ala Trp Leu Val Val Glu Ala Pro Gly
    690                 695                 700

Thr Gly Lys Glu Thr Ala Pro Glu Arg Arg Glu Val Val Thr Leu Arg
705                 710                 715                 720

Cys Asn His Arg Asp Ala Ser Met Leu Gly Ser Leu Ala Tyr Asn Val
                725                 730                 735

Leu Leu Ile Ala Leu Cys Thr Leu Tyr Ala Phe Lys Thr Arg Lys Cys
            740                 745                 750

Pro Glu Asn Phe Asn Glu Ala Lys Phe Ile Gly Phe Thr Met Tyr Thr
        755                 760                 765

Thr Cys Ile Ile Trp Leu Ala Phe Leu Pro Ile Phe Tyr Val Thr Ser
    770                 775                 780

Ser Asp Tyr Arg Val Gln Thr Thr Thr Met Cys Val Ser Val Ser Leu
785                 790                 795                 800

Ser Gly Ser Val Val Leu Gly Cys Leu Phe Ala Pro Lys Leu His Ile
                805                 810                 815

Ile Leu Phe Gln Pro Gln Lys Asn Val Val Ser His Arg Ala Pro Thr
            820                 825                 830

Ser Arg Phe Gly Ser Ala Ala Pro Arg Ala Ser Ala Asn Leu Gly Gln
        835                 840                 845

Gly Ser Gly Ser Gln Phe Val Pro Thr Val Cys Asn Gly Arg Glu Val
    850                 855                 860

Val Asp Ser Thr Thr Ser Ser Leu
865                 870

<210>  2
<211>  872
<212>  PRT
<213>  Homo sapiens

<400>  2